# EUROPEAN PATENT APPLICATION

(11) **EP 4 141 023 A1**
(43) Date of publication of application: **01.03.2023**
(21) Application number: 21792888.6
(22) Date of filing: 21.04.2021
(51) Int. Cl.: C07K 14/195, C12N 15/70, A61K 38/16, A61P 3/00

(54) **PROTEIN VARIANT HAVING GLP-1 INDUCING ACTIVITY AND USE THEREOF**

(30) Priority: 24.04.2020 KR 20200050338
(71) Applicant: Kobiolabs, Inc., Seoul 08826 (KR)
(72) Inventor: JO, Inseong, Seoul 08826 (KR); MOON, Sung Hyun, Seoul 08826 (KR); CHO, Bo-Ram, Seoul 08826 (KR); NAM, Tae-Wook, Seoul 08826 (KR)
(74) Representative: HGF
(86) International application number: PCT/KR2021/005036
(87) International publication number: WO 2021/215828

(57) **Abstract**

This disclosure relates to a protein variant having improved GLP-1 inducibility and/or thermal stability and enzyme resistance, and a pharmaceutical composition for reducing body weight and/or body fat, suppressing appetite or treating or preventing of metabolic diseases, comprising the same.

## Description

### Technical Field

This disclosure relates to a protein variant having improved GLP-1 inducibility and/or thermal stability and enzyme resistance, and a pharmaceutical composition for reducing body weight and/or body fat, suppressing appetite, or treating or preventing of metabolic diseases, comprising the same.

### Background Art

Glucagon-like peptide-1 (GLP-1), an appetite-regulating hormone, is a hormone secreted from the small intestine by ingestion of food, and regulate appetite by increasing satiety, and regulate blood glucose by inducing insulin secretion from the pancreas. GLP-1 is secreted from L-cells, a type of intestinal endocrine cells present in the ileum and colon.

GLP-1 is known to be useful in the treatment of various metabolic diseases, such as diabetes, obesity, heart disease, cerebrovascular disease, and arteriosclerosis. In addition, GLP-1 is involved in exhibiting the effect of treating diabetes by stimulating glucose-dependent insulin secretion in the pancreas, enhancing insulin gene expression, enhancing pancreatic beta cell proliferation, enhancing pancreatic beta cell survival, inhibiting glucagon secretion, and lowering blood glucose, and is involved in exhibiting the effect of treating obesity by slowing the emptying of the stomach, suppressing appetite, enhancing satiety, and suppressing food intake. In addition, it has been reported that it exhibits a therapeutic effect on heart disease through the protective effect of cardiomyocytes from ischemia and the enhancement effect of cardiac function in patients at risk of heart attack (Sokos, G.G. et al., Glucagon-like peptide-1 infusion improves left ventricular ejection fraction and functional status in patients with chronic heart failure., J. Card. Fail. (2006) 12: 694-699.; Ban, K., et al., Cardioprotective and vasodilatory actions of glucagon-like peptide-1 receptor are mediated through both glucagon-like peptide-1 receptor-dependent and -independent pathways, Circulation (2008) 117: 2340-2350).

Meanwhile, intestinal microorganisms are known to have a deep correlation with metabolic diseases such as obesity and diabetes, and in particular, the *Akkermansia muciniphila* strain is attracting attention as a therapeutic agent for metabolic disorders, including obesity, because it exhibits effects such as reducing plasma cholesterol when administered to mice on high-fat diet, increasing the subject's energy consumption, and inducing increased satiety and body weight loss (Korean Patent Publication No. 2015-0133646, Korean Patent Application Publication No. 2018-0053336, Korean Patent No. 1809172, etc.)

Accordingly, the present inventors have confirmed that *Akkermansia muciniphila* increases the UCP-1 factor affecting brown fat activity for the purpose of thermogenesis, and induces the expression of the appetite-regulating hormone GLP-1 in the small intestine using the reference strain of *Akkermansia muciniphila* (Akk; American Type Culture Collection, Deposit number: ATCC BAA-835) and the *Akkermansia muciniphila* SNUG-61027 strain isolated from healthy Korean feces (Deposit number: KCTC13530). In addition, the present inventors have found that the B2UM07 protein (Gene: Amuc_1631, Carboxyl-terminal protease) derived from the *Akkermansia muciniphila* strain SNUG-61027 promotes the induction of GLP-1 secretion (Korean Patent Application No. 2019-0125670).

Furthermore, the present inventors continued to study to improve the GLP-1 inducibility, thermal stability, and enzyme resistance of the B2UM07 protein. As a result, the present inventors constructed various variants of the B2UM07 protein and confirmed that they are useful for reducing weight and/or body fat, suppressing appetite, or treating or preventing of metabolic diseases based on their excellent GLP-1 inducibility, thermal stability and enzyme resistance, and thus completed the present invention.

### [Related Art Document]

### [Patent Document]

(Patent Document 1) Korean Patent Publication No. 2015-0133646
(Patent Document 2) Korean Patent Publication No. 2018-0053336
(Patent Document 3) Korean Patent No. 1809172

### Disclosure of Invention

### Technical Goals

Accordingly, an object of the disclosure is to provide a protein comprising the amino acid sequence of SEQ ID NO: 2 or SEQ ID NO: 4, and the protein variants comprising one or more amino acid substitutions in its amino acid sequences exhibiting at least one or more of excellent GLP-1 inducibility, thermal stability and enzyme resistance.

Another object of the disclosure is to provide a polynucleotide encoding the protein variant.

Another object of the disclosure is to provide a vector comprising the polynucleotide.

Another object of the disclosure is to provide a host cell comprising the vector.

Another object of the disclosure is to provide a pharmaceutical composition for reducing body weight and/or body fat, suppressing appetite, or treating or preventing metabolic diseases, comprising the protein variant and a pharmaceutically acceptable carrier.

Another object of the disclosure is to provide a food composition for reducing body weight and/or body fat, suppressing appetite, or improving or preventing metabolic diseases, comprising the protein variant.

### Technical Solutions

The present inventors have completed the present invention by constructing a protein variant that exhibits excellent effects in reducing body weight and/or body fat, suppressing appetite and treating, preventing, or improving various metabolic diseases by introducing various mutations into the B2UM07 protein to improve GLP-1 inducibility, thermal stability and enzyme resistance.

As used herein, the term "protein variant" refers to a variant including a variation in which one or more amino acids are substituted, added, or deleted in the amino acid sequence (SEQ ID NO: 1 below) of the full-length B2UM07 protein of the wild type. In the present specification, the amino acid number of the protein variant is indicated based on the amino acid number of the amino acid sequence (SEQ ID NO: 1 below) of the wild-type full-length B2UM07 protein.

[SEQ ID NO: 1]

<Akkermansia muciniphila SNUG-61027 (KBL983)B2UM07>

MEKNAPFSVMNMHSFRWIRLTAFSALAAAAITSCASAATDFNQVGKQMSLLLQNFHFSRKEFSDELSTKFLETYL

RKVDPNKIFFTQQDVDALKRKYGKELDDYLMSGQMMDAAQAMHALYRQRAMQRISYARDLLKKGGFTFDKDKSIE

RSRRKTAAWPKDEAEMQQVWKDMVEEQLLSEILRRETVARLAKEQNKPDPLANEKPAEEKLLMRYERIQRNIQET

DLEDVAETLLSAVALTYDPHTDYMGARQVDRFKISMGTELTGIGALLGSEDDGSTKITGIVVGGPADKSGELKLN

DRIVAIDSDNSGEMVDILFMKLDKVVDMIRGAENTQMRLKVEPADAPGQAKIITLTRSKVPLKDELAKGEIIELT

GAPEGRNRIGVLSLPSFYADMEGGDRRCAKDVKKILERMNKENVDGLVIDLRSNGGGSLEEVRLMTGFFTGNGPV

VQIKDTRGNVDIKSAHNRQKLFNGPIVVLINKLSASASEILAAALQDYGRAVIVGDESTFGKGSVQQPVDIGQYL

PFFAARDRAGLLKVTTQKFYRVAGGSTQLKGVESDIQLPTATAAFELGEDILDYAMPYDQITPCTNYKKDSSIAA

MLPVLKDASAKRVEKDRDLQIAREDIAMMKQRIKDNKLSLNKKIREQENSALEERRKSINKERKIRFAEMAKEDA

TKYKIYRLTLDDVNAKELPLADPEKDNEQFMHLAEDPTAELDDSPEYPSGLDPELREGINIVQDMLKLESSGK

According to a preferred embodiment of the disclosure, the disclosure relates to a protein variant including the amino acid sequence of SEQ ID NO: 2 below, in which 9 amino acids at the N-terminus are truncated from the amino acid sequence of the wild type full-length B2UM07 protein (SEQ ID NO: 1), and the hexa-histidine-tag is linked to the C-terminus. More preferably, the protein variant of the disclosure consists of the amino acid sequence of SEQ ID NO:2. The protein variant of SEQ ID NO: 2 is indicated by 10-748-His based on the amino acid number of the amino acid sequence of the wild-type full-length B2UM07 protein (SEQ ID NO: 1).

[SEQ ID NO: 2]

m nmhsfrwirl tafsalaaaa itscasaatd fnqvgkqmsl llqnfhfsrk
61 efsdelstkf letylrkvdp nkifftqqdv dalkrkygke lddylmsgqm mdaaqamhal
121 yrqramqris yardllkkgg ftfdkdksie rsrrktaawp kdeaemqqvw kdmveeglls
181 eilrretvar lakeqnkpdp lanekpaeek llmryeriqr niqetdledv aetllsaval
241 tydphtdymg arqvdrfkis mgteltgiga llgseddgst kitgivvggp adksgelkln
301 drivaidsdn sgemvdilfm kldkvvdmir gaentqmrlk vepadapgga kiitltrskv
361 plkdelakge iieltgapeg rnrigvlslp sfyadmeggd rrcakdvkki lermnkenvd
421 glvidlrsng ggsleevrlm tgfftgngpv vqikdtrgnv diksahnrqk lfngpivvli
481 nklsasasei laaalqdygr avivgdestf gkgsvqqpvd igqylpffaa rdragllkvt
541 tqkfyrvagg stqlkgvesd iqlptataaf elgedildya mpydqitpct nykkdssiaa
601 mlpvlkdasa krvekdrdlq iarediammk qrikdnklsl nkkireqens aleerrksin
661 kerkirfaem akedatkyki yrltlddvna kelpladpek dneqfmhlae dptaelddsp
721 eypsgldpel reginivqdm lklessgkHHHHHH

As one embodiment of the disclosure, the protein variant including the amino acid sequence of SEQ ID NO: 2 may be constructed by amplifying the DNA of SEQ ID NO: 3 encoding amino acids 10 to 748 of the B2UM07 protein with an appropriate primer pair by PCR in the Amuc_1631 gene, followed by cloning, protein expression and purification processes conventional in the art.

[SEQ ID NO: 3]

ATGAACATGCACTCATTCCGTTGGATTAGACTCACCGCATTCTCGGCCCTGGCCGCAGCCGCCATTACTTCCTGC
GCCTCTGCGGCTACGGACTTCAACCAGGTGGGCAAGCAAATGTCCCTGCTGCTCCAGAATTTCCACTTCTCCCGC
AAAGAATTCAGCGATGAACTATCCACTAAATTCCTGGAAACCTACCTGCGCAAGGTAGACCCCAACAAAATATTC
TTCACCCAGCAGGACGTAGACGCCCTCAAAAGAAAATACGGTAAGGAGCTGGACGACTACCTTATGTCCGGCCAG
ATGATGGATGCGGCCCAGGCCATGCACGCCCTTTACCGCCAGCGCGCCATGCAGCGCATCTCCTATGCGCGGGAT
TTGCTGAAAAAGGGAGGCTTCACCTTTGACAAAGACAAGTCTATCGAACGTTCCCGCCGCAAAACAGCCGCGTGG
CCCAAGGATGAGGCGGAAATGCAGCAGGTCTGGAAAGACATGGTGGAGGAACAGCTCCTGTCCGAAATCCTGCGC
CGTGAAACCGTAGCGCGCCTGGCCAAGGAACAGAACAAGCCCGATCCCCTGGCCAATGAAAAACCCGCGGAGGAA
AAACTGCTTATGCGTTATGAACGCATTCAGCGCAATATTCAGGAAACGGATCTGGAAGACGTAGCGGAAACACTG
CTCAGCGCCGTAGCCTTGACGTATGACCCGCATACGGATTACATGGGTGCGCGCCAAGTGGACCGTTTCAAAATC
TCCATGGGTACGGAACTCACCGGCATCGGCGCCCTGTTGGGCAGTGAAGACGACGGTTCCACCAAAATTACCGGT
ATCGTTGTGGGAGGGCCGGCTGACAAATCCGGAGAATTGAAGCTGAACGACCGCATCGTTGCCATTGACTCCGAC
AACTCTGGAGAAATGGTGGATATCCTGTTCATGAAGCTGGACAAAGTGGTGGATATGATCCGCGGAGCCGAAAAT
ACCCAGATGCGCCTGAAAGTAGAGCCGGCAGACGCCCCTGGACAGGCCAAAATCATTACGCTGACCCGCTCCAAG
GTACCTCTGAAGGATGAACTTGCCAAAGGTGAAATCATTGAACTTACCGGAGCTCCGGAAGGCAGGAACCGCATT
GGCGTGCTGAGCCTTCCCTCCTTCTACGCAGACATGGAAGGCGGAGACCGCCGCTGTGCCAAGGATGTCAAAAAA
ATCCTGGAACGGATGAACAAGGAAAATGTGGATGGCCTGGTAATTGACCTGCGCAGCAACGGCGGCGGTTCCCTG
GAGGAAGTGCGCCTGATGACGGGCTTCTTTACCGGAAACGGCCCCGTGGTGCAAATCAAGGACACCCGCGGCAAC
GTGGATATCAAATCCGCCCACAACCGCCAGAAACTCTTCAATGGCCCCATTGTGGTGCTCATCAATAAACTCAGC
GCATCCGCCTCTGAAATTCTGGCTGCGGCCCTTCAGGATTACGGCCGCGCCGTGATTGTGGGGGATGAATCCACC

TTCGGGAAGGGGTCTGTGCAGCAGCCTGTGGACATCGGCCAATACCTGCCTTTCTTCGCGGCCAGAGACCGTGCG
GGCCTGCTGAAAGTCACTACCCAGAAATTTTACCGTGTGGCGGGCGGCTCCACCCAGCTCAAAGGCGTGGAAAGC
GATATCCAGCTTCCCACCGCTACGGCGGCATTCGAACTGGGAGAAGACATTCTGGACTACGCGATGCCCTATGAC
CAGATTACGCCCTGCACCAACTACAAAAAGGACTCCTCCATCGCGGCCATGCTGCCCGTGCTGAAAGATGCCAGC
GCGAAGCGCGTGGAAAAAGACCGCGACCTCCAGATTGCCAGGGAAGATATCGCCATGATGAAACAGCGCATCAAG
GACAACAAGCTTTCCCTGAACAAGAAAATCCGGGAACAGGAAAACTCCGCCCTGGAAGAACGCCGCAAATCCATC
AACAAGGAACGTAAAATCCGCTTCGCGGAAATGGCCAAGGAAGACGCTACCAAATACAAAATTTACCGCCTGACG
CTGGACGACGTCAACGCCAAGGAGCTGCCCCTGGCGGATCCGGAAAAAGACAATGAACAATTCATGCACCTGGCG
GAAGACCCCACGGCAGAACTGGACGACTCCCCGGAATACCCCTCCGGCCTTGATCCGGAACTCCGCGAAGGCATC
AACATCGTCCAGGATATGCTGAAGCTGGAATCCTCCGGAAAA

In addition, as another aspect of the disclosure, the disclosure relates to a protein variant including the amino acid sequence of SEQ ID NO: 4, characterized in that the hexa-histidine-tag at the C-terminus of the amino acid sequence of SEQ ID NO: 2 is inserted between the amino acids 37 and 38. The protein variant of SEQ ID NO: 4 is indicated by (10-37)-His-(38-748) based on the amino acid number of the amino acid sequence of the wild-type full-length B2UM07 protein (SEQ ID NO: 1).

[SEQ ID NO: 4]

MNMHSFRWIRLTAFSALAAAAITSCASAHHHHHHATDFNQVGKQMSLLLQNFHFSRKEFSDELSTKFLETYLRKV

DPNKIFFTQQDVDALKRKYGKELDDYLMSGQMMDAAQAMHALYRQRAMQRISYARDLLKKGGFTFDKDKSIERSR

RKTAAWPKDEAEMQQVWKDMVEEQLLSEILRRETVARLAKEQNKPDPLANEKPAEEKLLMRYERIQRNIQETDLE

DVAETLLSAVALTYDPHTDYMGARQVDRFKISMGTELTGIGALLGSEDDGSTKITGIVVGGPADKSGELKLNDRI

VAIDSDNSGEMVDILFMKLDKVVDMIRGAENTQMRLKVEPADAPGQAKIITLTRSKVPLKDELAKGEIIELTGAP

EGRNRIGVLSLPSFYADMEGGDRRCAKDVKKILERMNKENVDGLVIDLRSNGGGSLEEVRLMTGFFTGNGPVVQI

KDTRGNVDIKSAHNRQKLFNGPIVVLINKLSASASEILAAALQDYGRAVIVGDESTFGKGSVQQPVDIGQYLPFF

AARDRAGLLKVTTQKFYRVAGGSTQLKGVESDIQLPTATAAFELGEDILDYAMPYDQITPCTNYKKDSSIAAMLP

VLKDASAKRVEKDRDLQIAREDIAMMKQRIKDNKLSLNKKIREQENSALEERRKSINKERKIRFAEMAKEDATKY

KIYRLTLDDVNAKELPLADPEKDNEQFMHLAEDPTAELDDSPEYPSGLDPELREGINIVQDMLKLESSGK

In addition, as another aspect of the disclosure, the disclosure relates to a protein variant including at least one or more of the amino acid substitutions of Table 1 below in the amino acid sequence of SEQ ID NO: 4. The amino acid numbers described in Tables 1 to 4 below is indicated based on the amino acid number of the amino acid sequence of the wild-type full-length B2UM07 protein (SEQ ID NO: 1).

**[Table 1]**

| | |
|---|---|
| - C34A, C34S | - C403A, C403T |
| - S36H | - M414F, M414I |
| - A37H | - V423I |
| - G45A, G45L, G45V | - R438V, R438A, R438Q, R438N, R438H |
| - K46A, K46Q, K46N, K46H, K46T | - T445I |
| - R59A, R59Q, R59N, R59H | - G446R |
| - K60A, K60Q, K60N, K60H, K60T | - A465D |
| - V78L | - N467D |
| - K82A, K82Q, K82N, K82H, K82V, K82T | - K482A, K482Q, K482N, K482H, K482T |
| - K94A, K94Q, K94N, K94H, K94T | - S486A |
| - R95A, R95Q, R95N, R95H | - A492L, A492V |
| - K96A, K96Q, K96N, K96H, K96T | - A495I |
| - M213Q, M213V, M213I, M213A | - R500A |
| - A231F | - A501I, A501V |
| - A251P | - K512A, K512Q, K512N, K512H, K512T |
| - T263I, T263L, T263V | - R531A, R531Q, R531N, R531H |
| - D307A | - A534F, A534V |
| - K324D, K324N, K324A, K324Q, K324N, K324H, K324T | - K555R, K555A, K555Q, K555N, K555H |
| - K368A, K368Q, K368N, K368H, K368T | - Y583F, Y583V, Y583I, Y583A, Y583H |
| - G369A | - C589A, C589S, C589T |
| - R402S, R402A, R402Q, R402N, R402H | - G733A, G733V |

Preferably, the amino acid substitution may include at least one or more of the amino acid substitutions of Table 2 below.

**[Table 2]**

| | |
|---|---|
| - C34A, C34S | - C403A, C403T |
| - S36H | - M414F, M414I |
| - A37H | - V423I |
| - G45A, G45L, G45V | - R438V |
| - K46A | - T445I |
| - R59A | - G446R |
| - K60A | - A465D |
| - V78L | - N467D |
| - K82A | - K482A |
| - K94A | - S486A |
| - R95A | - A492L, A492V |
| - K96A | - A495I |
| - M213Q | - R500A |
| - A231F | - A501I, A501V |
| - A251P | - K512A |
| - T263I, T263L, T263V | - R531A |
| - D307A | - A534F, A534V |
| - K324D, K324N | - K555R, |
| - K368A | - Y583F, |
| - G369A | - C589A, C589S, C589T |
| - R402S | - G733A, G733V |

Preferably, the protein variant of the disclosure includes the amino acid sequence of SEQ ID NO: 4, and may include one amino acid substitution of the amino acid substitutions of Table 2. In addition, the protein variant of the disclosure includes the amino acid sequence of SEQ ID NO: 4, and may include 2 to 5 amino acid substitutions of the amino acid substitutions of Table 2. In this case, the 2 to 5 amino acid substitutions may preferably include one or more amino acid substitutions of K368A, C403A and C589A.

More preferably, the protein variant of the disclosure may include any one of the amino acid substitutions of Table 3 below.

**[Table 3]**

| | | | | |
|---|---|---|---|---|
| C34A | A251P | R402S | V423I | A501V |
| C34S | T263I | R402S/Y583F | R438V | K512A |
| S36H/A37H | T263L | C403A | R438V/G446R | R531A |
| G45A | T263V | C403A/C589A | T445I | A534F |
| G45L | D307A | C403A/C589A/S36H/A37H | A465D | C589A |
| G45V | K324D | C403A/C589A/K46A | N467D | C589A/C34S |
| K46A | K324D/A534V | C403A/C589A/K82A | K482A | C589A/S36H/A37H |
| R59A/K60A | K324N | C403A/C589A/K94A/R95A/K96A | S486A | C589S |
| V78L | K368A | C403A/C589A/K368A | A492L | C589T |
| K82A | K368A/K46A | C403T | A492V | G733A |
| K94A/R95A/K96A | K368A/K82A | M414F | A495I | G733V |
| M213Q | K368A/K94A/R95A/K96A | M414! | R500A | |
| A231F | G369A | M414I/K555R | A501I | |

More preferably, the protein variant of the disclosure includes the amino acid sequence of SEQ ID NO: 4, and may include the C403A/C589A amino acid substitution, and may further include at least one or more of the amino acid substitutions of Table 4 below.

**[Table 4]**

| | |
|---|---|
| - Q43C | - E379C |
| - R59C | - K409C |
| - R95C | - K416C |
| - K99C | - S464C |
| - S107C | - R468C |
| - A119C | - Q523C |
| - K138C | - A530C |
| - T142C | - Q562C |
| - A158C | - D575C |
| - K161C | - Q585C |
| - A164C | - T590C |
| - A189C | - S596C |
| - K193C | - A610C |
| - N203C | - A627C |
| - R220C | - K634C |
| - K258C | - A651C |
| - S311C | - S658C |
| - A332C | - K700C |
| - S358C | -E710C |
| - K363C | - S745C |

The protein variant of the disclosure including the amino acid sequence of SEQ ID NO: 4, and including the amino acid substitutions described in Tables 1 to 3, and the amino acid substitutions described in Table 4 together with the C403A/C589A amino acid substitution, may have increased one or more of GLP-1 inducibility, thermal stability and enzyme resistance compared to the protein variant including the amino acid sequence of SEQ ID NO: 2. The protein variant of the disclosure including the amino acid substitutions described in Tables 1 to 3, and the amino acid substitutions described in Table 4 together with the C403A/C589A amino acid substitution also include within the scope of this disclosure functional equivalents or functional derivatives having a functionally substantially equivalent activity thereto. Specifically, the protein variant of the disclosure including the amino acid substitutions described in Tables 1 to 3, and the amino acid substitutions described in Table 4 together with the C403A/C589A amino acid substitution may exhibit increased GLP-1 inducibility compared to the protein including the amino acid sequence of SEQ ID NO: 2 by about 10% or more, about 20% or more, about 30% or more, about 40% or more, about 50% or more, about 60% or more, or about 70% or more.

In addition, the protein variant of the disclosure including the amino acid substitutions described in Tables 1 to 3, and the amino acid substitutions described in Table 4 together with the C403A/C589A amino acid substitution may exhibit improved thermal stability by having an increased melting temperature (Tm) of about 0.2 °C or more, about 0.5 °C or more, about 0.8 °C or more, about 1 °C or more or about 1.5 °C compared to the protein including the amino acid sequence of SEQ ID NO: 2.

In addition, the protein variant of the disclosure including the amino acid substitutions described in Tables 1 to 3, and the amino acid substitutions described in Table 4 together with the C403A/C589A amino acid substitution may exhibit improved stability, that is, enzyme resistance against degradation by digestive enzymes such as trypsin and chymotrypsin.

As used herein, the term "enzyme resistance" may indicate to stability against degradation by digestive enzyme in vitro or in vivo. Digestive enzyme resistance according to the disclosure can be measured through conventional means and methods known in the art.

In one embodiment of the disclosure, the disclosure relates to a polynucleotide encoding a protein variant including the amino acid sequence of SEQ ID NO: 2 or SEQ ID NO: 4, or encoding a protein variant including amino acid substitutions described in Tables 1 to 3 in the amino acid sequence of SEQ ID NO: 4, and the amino acid substitutions described in Table 4 together with the C403A/C589A amino acid substitution.

The polynucleotide encoding the protein variant of the disclosure may be variously modified in the coding region within a range that does not change the amino acid sequence of the protein variant according to the disclosure due to the degeneracy of codons or in consideration of codons preferred in organisms intended to express the protein variant of the disclosure, and may be variously changed or modified within the range that does not affect the expression of the protein in parts other than the coding region. And such modified polynucleotides are included in the scope of the disclosure. A polynucleotide encoding a protein variant of the disclosure may be provided by a vector expressing it.

In one embodiment of the disclosure, the disclosure relates to a vector including a polynucleotide encoding a protein variant of the disclosure.

As used herein, "vector" refers to a means for expressing a protein variant by introducing a polynucleotide encoding the protein variant of the disclosure into a host cell. The vector of the disclosure includes all conventional vectors including plasmids, cosmids and viruses (for example, bacteriophages), and is preferably a plasmid vector. Those skilled in the art can construct vectors by standard recombinant techniques.

The vector of the disclosure may include various regulatory sequences. Vectors and expression vectors may also include nucleic acid sequences that provide other functions as well as regulatory sequences that regulate transcription and translation,.

In one embodiment of the disclosure, the disclosure relates to a host cell including the vector.

As used herein, the term "host cell" includes eukaryotes and prokaryotes, and refers to any transformable organism capable of replicating the vector or expressing genes encoded by the vector. A host cell may be transfected or transformed by the vector, which refers to a process in which an exogenous nucleic acid molecule is delivered or introduced into a host cell.

According to a preferred embodiment of the disclosure, the host cell of the disclosure is a bacterial cell, more preferably a gram-negative bacterial cell, still more preferably *E. coli.*

In another aspect of the disclosure, the disclosure relates to a pharmaceutical composition including a protein variant including the amino acid sequence of SEQ ID NO: 2 or SEQ ID NO: 4, or a protein variant including amino acid substitutions described in Tables 1 to 3 in the amino acid sequence of SEQ ID NO: 4, and the amino acid substitutions described in Table 4 together with the C403A/C589A amino acid substitution, and a pharmaceutically acceptable carrier.

Preferably, the pharmaceutical composition of the disclosure is useful for reducing body weight and/or body fat, suppressing appetite, or treating or preventing metabolic diseases.

In the disclosure, the metabolic diseases include, but is not limited to, metabolic syndrome, obesity, insulin resistance, glucose tolerance, diabetes, arteriosclerosis, dyslipidemia, hyperlipidemia, hypercholesterolemia, fatty liver, cardiovascular disease, hypertension and osteoporosis.

In the disclosure, "treatment" means preventing (preventing from occurring), reducing or alleviating at least one of the symptoms or adverse effects of a disease, disorder or condition. As used herein, "treatment" refers to both therapeutic treatment and prophylactic or preventative measures, and is intended to prevent or slow down (ameliorate) a targeted pathological condition or disorder. In one embodiment of the disclosure, a subject in need of treatment includes not only a subject already suffering from a disease or disorder, but also a subject most likely to have the disease or disorder or for whom the disease or disorder is to be prevented.

The pharmaceutical composition of the disclosure may be prepared in a unit dosage form by formulating using a pharmaceutically acceptable carrier and/or excipient according to a method that can be easily carried out by a person of ordinary skill in the art, or may be prepared by incorporation into a multi-dose container. In this case, the formulation may be a solution in an oil or aqueous medium, suspension, or emulsion, or may be in the form of an extract, powder, granule, tablet or capsule, and may additionally include a dispersant or stabilizer. Formulations for parenteral use may be sterilized, and non-limiting examples of sterilization techniques include filtration through bacteriostatic filters, terminal sterilization, incorporation of sterile agents, irradiation, heating, vacuum drying and freeze drying.

Pharmaceutically acceptable carriers included in the pharmaceutical composition of the disclosure are commonly used in formulation, and includes, but is not limited to, lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methylhydroxybenzoate, propylhydroxybenzoate, talc, magnesium stearate and mineral oil. The pharmaceutical composition of the disclosure may further include, in addition to the above components, lubricants, wetting agents, sweetening agents, flavoring agents, emulsifying agents, suspending agents, preservatives, antiseptics, buffering agents (for example, acetic acid, citric acid, phosphoric acid in salt form etc.). In addition, the pharmaceutical composition of the disclosure may be formulated in a freeze-dried formulation.

The pharmaceutical composition of the disclosure may be administered orally or parenterally to a subject, for example, may be administered by oral administration, intravenous injection, local injection, intraperitoneal injection, and the like.

A suitable dosage of the pharmaceutical composition of the disclosure varies depending on factors such as formulation method, administration mode, patient's age, weight, sex, pathological condition, food, administration time, administration route, excretion rate, and response sensitivity, and a skilled physician can easily determine and prescribe an effective dosage for the desired treatment or prevention. According to a preferred embodiment of the disclosure, the daily dose of the pharmaceutical composition of the disclosure is 0.0001-100 mg/kg.

The pharmaceutical composition of the disclosure may be administered in combination with an additional agent commonly used for the purpose of reducing body weight and/or body fat, suppressing appetite, or treating or preventing metabolic diseases.

In another aspect of the disclosure, the disclosure relates to a food composition including a protein variant including the amino acid sequence of SEQ ID NO: 2 or SEQ ID NO: 4, or a protein variant including amino acid substitutions described in Tables 1 to 3 in the amino acid sequence of SEQ ID NO: 4, and the amino acid substitutions described in Table 4 together with the C403A/C589A amino acid substitution.

The food composition of the disclosure may be useful for reducing body weight and/or body fat, suppressing appetite, or improving or preventing of metabolic diseases.

The food composition of the disclosure can be easily utilized as a food effective for reducing body weight and/or body fat, suppressing appetite, or improving or preventing of metabolic diseases, such as a main raw material, a supplementary raw material, a food additive, a health functional food or a functional beverage of food, but is not limited thereto.

The food composition means a natural product or processed product containing one or more nutrients, and preferably means a state that can be eaten directly through a certain amount of processing.

When the composition of the disclosure is provided in the form of a food composition, the composition of the disclosure may include, in addition to the active ingredient, ingredients commonly added during food production, for example, protein, carbohydrate, fat, nutrients, seasoning and flavoring agents.

The food composition according to the disclosure may be produced using a method known in the art, and may contain the same weight of the protein variant as the pharmaceutical composition of the disclosure. For example, the food composition according to the disclosure may include the protein variant of the disclosure in an amount of 0.001% to 100% by weight, preferably 1% to 99% by weight of the total food weight, and in the case of a beverage, the protein variant may be included in a ratio of 0.001 g to 10 g, preferably 0.01 g to 1 g per 100 mL. The amount of protein variants in food may depend on a variety of factors, including the capacity of the food, frequency of consumption of the food, the type of strain contained in the food, the amount of moisture in the food, and/or additional conditions for the survival of the strain in the food.

In another aspect of the disclosure, a method of reducing body weight and/or body fat, suppressing appetite, or treating or preventing metabolic diseases is provided, and the method includes administering a pharmaceutically effective amount of a protein variant according to this disclosure to a subject in need of weight and/or body fat reduction, appetite suppression, or treatment or prevention of metabolic diseases.

The subject to the prevention or treatment of the diseases includes all animals including humans. For example, it may be an animal such as a dog, a cat, or a mouse, preferably a human.

In another aspect of the disclosure, the use of a protein variant or composition of the disclosure for use in reducing body weight and/or body fat, suppressing appetite, or treating or preventing metabolic diseases, and the use of a protein variant or composition of the disclosure for the manufacture of a medicament for reducing body weight and/or body fat, suppressing appetite, or treating or preventing metabolic diseases is provided.

Since the pharmaceutical composition and administration method used in the method for preventing or treating the diseases are the same as those described above, descriptions of common contents between the two will be omitted.

The disclosure is not limited in its application to the details of the arrangement and construction of components set forth in the following description or illustrated in the drawings. The disclosure is capable of other embodiments and of being practiced or carried out in various ways. Also, the phraseology and terminology used herein is for the purpose of description and should not be regarded as limiting. The use of "comprise", "comprising" or "having", "containing", "comprised" and variations thereof herein is meant to encompass the items listed thereafter and equivalents thereof, as well as additional items.

Unless defined otherwise herein, scientific and technical terms used in connection with the disclosure should have the meanings commonly understood by one of ordinary skill in the art. The methods and techniques of the disclosure are generally performed according to conventional methods well known in the art. In general, the nomenclature and techniques used in connection with biochemistry, enzymology, molecular and cell biology, microbiology, virology, cell or tissue culture, genetics, and protein and nuclear chemistry described herein are well known in the art and commonly will be used. The methods and techniques of the disclosure are generally performed in accordance with conventional methods well known in the art, and performed as described in various general methods or referenced guidelines or protocols cited and discussed throughout this specification, unless otherwise indicated.

### Effects

The protein variant of the disclosure exhibits excellent GLP-1 inducibility, improved thermal stability and enzyme resistance, and thus can be usefully utilized for reducing body weight and/or body fat, suppressing appetite, or treating or preventing metabolic diseases.

### Brief Description of Drawings

FIG. 1 shows the results of measuring the GLP-1 inducibility of protein variant P9-00(1-748-His), P9-01(10-748-His) and P9-02((10-37)-His-(38-748)).
FIG. 2 shows the results of observing the GLP-1 expression level of P9-02-based variants by ELISA as a relative ratio to the GLP-1 expression level of P9-02.
FIG. 3 is a result of observing the increase and decrease of the protein melting temperature (Tm) of the P9-02-based variants by real-time PCR compared to the protein melting temperature of P9-02.
FIG. 4 shows the SDS-PAGE results of PEGylated variant P9-2CA(P9-02((10-37)-His-(38-748)) C403A/C589A) and 40 kinds of P9-2CA-based variants.
FIG. 5 shows the results of measuring the GLP-1 inducibility of PEGylated P9-2CA-based variants.

### Best Mode for Carrying Out the Invention

Hereinafter, the disclosure will be described in more detail through examples. These examples are only for illustrating the invention, and it will be apparent to those of ordinary skill in the art that the scope of the invention is not to be construed as being limited by these examples.

### Example 1: Construction of full-length P9-based variants and comparison of efficacy

### 1-1: Cloning of the full-length B2UM07 protein tagged with 6 histidines at the C-terminus (P9-00(1-748-His) (SEQ ID NO: 113))

To construct pET26b::P9-00 (1-748-His), the first intermediate (5Kb) obtained from pET26b::P9-01(10-748-His) constructed in Example 1-2 using the primers of SEQ ID NO: 5 and SEQ ID NO: 6, and the second intermediate (2Kb) obtained from A. *muciniphila* genomic DNA (using Wizard Genomic DNA purification kit, Promega) using the primers of SEQ ID NO: 7 and SEQ ID NO: 8 were amplified using DNA polymerase (pfu, SPD16-R250, SolGent), respectively. The amplified first and second products were treated with Dpnl enzyme (FastDigest DpnI, ThermoFisher Scientific), and then synthesized as a single plasmid using Gibson Assembly Master Mix (E2611S, New England Biolab). After synthesis, the plasmid was transformed into DH5α-competent cells and plated on LB plates. Plasmids were extracted from a single colony, and mutations were confirmed through sequencing, and stored at -20 °C until use.

Table 5 below shows the primer sequences of SEQ ID NOs: 5 to 8.

**[Table 5]**

| | |
|---|---|
| pET26b_P9-his_F | 5'-ttaactttaagaaggagatatacatatggaaaaaaacgcacccttttccgttatg-3'(SEQ ID NO: 5) |
| pET26b R | 5'-ttagcagccggatctcagtggtggtggtggtggtgctcgagttttccggaggattccagcttcagcat- 3'(SEQ ID NO: 6) |
| pET_P9_l_F | 5'-cctccggaaaactcgagcaccaccaccaccaccactgagatccggctgctaacaaagccc-3'(SEQ ID NO: 7) |
| pET_P9_748-his_R | 5'-atgtatatctccttcttaaagttaaacaaaattatttctagaggggaatt-3'(SEQ ID NO: 8) |

### 1-2: Cloning of P9-01(10-748-His) (SEQ ID NO: 2)

DNA encoding amino acids 10 to 748 of the B2UM07 protein (SEQ ID NO: 3) was amplified by PCR in the Amuc_1631 gene with a pair of primers including Ndel and XhoI restriction enzyme site sequences. The pET26b(+) vector (Novogene, USA) to be used as an expression vector and the amplified PCR product were treated with Ndel and XhoI restriction enzymes and purified. After ligation of them, they were transformed into DH5α competent cells and plated on LB plates. Plasmids were extracted from a single colony, and mutations were confirmed through sequencing, and stored at -20 °C until use.

### 1-3: Cloning of P9-02((10-37)-His-(38-748)) (SEQ ID NO: 4)

To construct pET26b::P9-02((10-37)-His-(38-748)), the first intermediate (5Kb) obtained from pET26b::P9-01(10-748-His) constructed in Example 1-2 using the primers of SEQ ID NO: 9 and SEQ ID NO: 12, and the second intermediate (2Kb) obtained from *A. muciniphila* genomic DNA (using Wizard Genomic DNA purification kit, Promega) using the primers of SEQ ID NO: 10 and SEQ ID NO: 11 were amplified using DNA polymerase (pfu, SPD16-R250, SolGent), respectively. The amplified first and second products were treated with Dpnl enzyme (FastDigest Dpnl, ThermoFisher Scientific), and then synthesized as a single plasmid using Gibson Assembly Master Mix (E2611S, New England Biolab). After synthesis, the plasmid was transformed into DH5α-competent cells and plated on LB plates. Plasmids were extracted from a single colony, and mutations were confirmed through sequencing, and stored at -20 °C until use.

Table 6 below shows the primer sequences of SEQ ID NOs: 9 to 12.

**[Table 6]**

| | |
|---|---|
| pET_P9_ΔHis_F | 5'-agctggaatcctccggaaaatgagatccggctgctaacaaagccc-3'(SEQ ID NO: 9) |
| pET_P9_ ΔHis_R | 5'-ttgttagcagccggatctcattttccggaggattccagcttcagca-3'(SEQ ID NO: 10) |
| pET_P9(His-38)_F | 5'-caccaccaccaccaccacgctacggacttcaaccaggtgggc-3'(SEQ ID NO: 11) |
| pET_P9(His-38)_R | 5'-gtggtggtggtggtggtgcgcagaggcgcaggaagtaatggc-3'(SEQ ID NO: 12) |

### Example 2: Expression and purification of full-length P9-based variants

To prepare LB medium, ~400 ml DW and a magnetic bar were placed in a clean beaker. 12.5 g of LB broth (BD Difco^{™} LB broth, Miller) was placed in a beaker and dissolved while stirring. The solution was transferred to a measuring cylinder and the volume was adjusted to 500 ml using DW. 500 ml of the solution was transferred to a 1 L flask, the top of the flask was covered with aluminum foil, and the flask was sterilized by autoclaving (121 °C, 15 minutes). Cool to below 40 °C and add 500 µl of 40 mg/ml kanamycin stock prior to use.

Each expression plasmid suitable for expression of P9-00, P9-01 and P9-02 obtained in Example 1 was transformed into *E. coli,* and a single or a number of colonies of the transformed *E. coli* carrying each expression plasmid were inoculated into 25 ml LB medium (supplemented with 40 µg/ml kanamycin). It was incubated at 30 °C with shaking at 200 rpm until OD₆₀₀ reached ~1.0.

The 25 ml LB medium inoculated with the bacteria was transferred to a 1 L flask containing 500 ml LB medium. It was incubated at 30 °C with shaking at 200 rpm until the OD₆₀₀ reached 0.7-0.8. To induce protein expression, 250 µL of 1 M isopropyl β-D-1-thiogalactopyranoside (IPTG) stock was added and incubated at 30 °C with shaking at 200 rpm for 4 h. Thereafter, the cells were harvested by centrifugation (6000 g, 20 minutes). Cell pellets were stored at -80 °C until use.

For the preparation of cell lysates of *E. coli* for Ni-NTA chromatography, the harvested cell pellets was resuspended in 40 ml of lysis buffer (1X PBS supplemented with 2 mM 2-mercaptoethanol (20X PBS dilution, BIOSESANG)), and transferred to a 100 ml glass beaker. The probe of the sonicator was placed 1 cm above the bottom of the beaker. Cell suspensions were sonicated on ice for 8 min by 240 repetitions of short pulses of 20 kHz per 1 s (pulse on, 45 µm amplitude) and 1 s intervals (pulse off) (i.e., pulse ON: OFF = 1 s: 1 s, 45 µm amplitude, 8 min treatment; suspension is always kept on ice). Then, it was cooled on ice for 8 minutes and the sonication was repeated. Then, cell debris was removed by centrifugation at 36,000 g for 20 minutes at 4 °C.

To perform Ni-NTA chromatography, 0.5 ml of settled Ni-NTA resin (HisPur Ni-NTA resin, ThermoFisher) was added to a 60 ml open column (manufactured by Bio-Rad). The resin was washed with 1 column volume (CV) of DW and equilibrated with 2 CV of 1X PBS. The column was loaded with the prepared cell lysate, and the column was rotated at 4 °C for 30 minutes. The resin was washed with 200 ml of wash buffer containing 20 mM Tris-HCl (pH 8.0), 150 mM NaCl, 20 mM imidazole and 2 mM 2-mercaptoethanol. The His-tagged protein was then eluted with 1 ml of elution buffer containing 20 mM Tris-HCl (pH 8.0), 150 mM NaCl, 200 mM imidazole and 2 mM 2-mercaptoethanol. This operation was repeated 10 times and each fraction was collected in a separate tube. The fractions were analyzed by SDS-PAGE.

For dialysis of protein samples, 1-5 fractions containing the target protein were collected. The dialysis cassette (ThermoFisher Scientific Slide-A-Lyzer Dialysis Cassette (10K MWCO)) was hydrated with 1xPBS for 5 to 10 min. The collected protein was injected into a hydrated dialysis cassette using a 10 ml syringe. A beaker was filled with cold 1xPBS buffer to at least 100 times the sample volume, and a magnetic bar for stirring was placed. A dialysis cassette containing a protein sample was added to this beaker, and then stirred at 4 °C for 2 hours. The beaker was refilled with fresh 1x PBS buffer and stirred overnight at 4 °C. Samples were obtained using a 10 ml syringe. The dialyzed protein samples were kept frozen at -80 °C in aliquots of 100 µL.

### Example 3: GLP-1 expression assay of full-length P9-based variants

### 3-1: NCI-H716 cell culture for in vitro assays and ELISA test

NCI-H716 (ATCC^{®} CCL-251^{™}), a Human L cell (cell line) in the human intestine, was acquired from ATCC and used in this experiment. As the culture media, RPMI1640 (welgene; LM 011-51), 10% FBS, and 1% Penicillin-Streptomycin were mixed and used. After subculture of NCI-H716 cells in the culture media (Cell passage number is less than 30), it was inoculated with 2×10⁴ to 6×10⁴ cells/200 µl in a collagen-coated 96 well plate, and used after culture for 1 day.

After removing the culture media from the plate, 200 µl of the protein variants P9-00, P9-01 and P9-02 cloned, expressed and purified in Examples 1 and 2 were treated 3 times in each well at a concentration of 0.05-0.1 mg/ml, and after 2 hours of incubation, the supernatant was separated and analyzed by ELISA to confirm GLP-1 inducibility. For ELISA analysis, RayBio^{®} Human/Mouse/Rat GLP-1 Enzyme Immunoassay kit was used and the experiment was performed according to the recommended protocol of the kit.

### 3-2: Comparison of GLP-1 inducibility of full-length P9-based variants

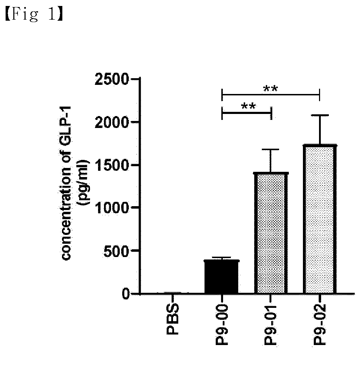

As a result of measuring the GLP-1 inducibility of protein variants P9-00, P9-01 and P9-02 according to Example 3-1, P9-01 exhibited approximately 3.5-fold increased GLP-1 inducibility than P9-00, and P9-02 exhibited a GLP-1 inducibility that was increased by 20% or more compared to P9-01 (see FIG. 1).

### Example 4: Preparation of additional P9-02((10-37)-His-(38-748))-based variants

Based on P9-02, which showed the best GLP-1 inducibility in Example 3-2, additional variants were constructed using a whole plasmid site-directed mutagenesis method. The entire pET26b::P9-02((10-37)-His-(38-748)) plasmid encoding the P9-02 protein constructed in Example 1-3 amplified by PCR using a complementary mutagenic primer pair shown in Table 6 for each variant. High-fidelity PfuDNA polymerase (nPfu-forte, Enzynomics) was used. The amplified PCR product was treated with Dpnl enzyme (FastDigest Dpnl, ThermoFisher Scientific), then transformed into DH5α-competent cells and plated on LB plates. Plasmids were extracted from a single colony, and mutations were confirmed through sequencing, and stored at -20 °C until use.

Table 7 below shows the primer sequences for site-directed mutagenesis of SEQ ID NOs: 13 to 112.

**[Table 7]**

| | |
|---|---|
| P9_G45A_F | 5'-gacttcaaccaggtggccaagcaaatgtccctg-3' (SEQ ID NO: 13) |
| P9_G45A_R | 5'-cagggacatttgcttggccacctggttgaagtc-3' (SEQ ID NO: 14) |
| P9_G45V_F | 5'-gacttcaaccaggtggtcaagcaaatgtccctg-3' (SEQ ID NO: 15) |
| P9_G45V_R | 5'-cagggacatttgcttgaccacctggttgaagtc-3' (SEQ ID NO: 16) |
| P9_G45L_F | 5'-tacggacttcaaccaggtgctaaagcaaatgtccctgctgc-3' (SEQ ID NO: 17) |
| P9_G45L_R | 5'-gcagcagggacatttgctttagcacctggttgaagtccgta-3' (SEQ ID NO: 18) |
| P9_T263I_F | 5'-tcaaaatctccatgggtatagaactcaccggcatcggc-3' (SEQ ID NO: 19) |
| P9_T263I_R | 5'-gccgatgccggtgagttctatacccatggagattttga-3' (SEQ ID NO: 20) |
| P9_T263V_F | 5'-cgtttcaaaatctccatgggtgtggaactcaccggcatc-3' (SEQ ID NO: 21) |
| P9_T263V_R | 5'-gatgccggtgagttccacacccatggagattttgaaacg-3' (SEQ ID NO: 22) |
| P9_V423I_F | 5'-aaaatgtggatggcctgataattgacctgcgcagc-3' (SEQ ID NO: 23) |
| P9_V423I_R | 5'-gctgcgcaggtcaattatcaggccatccacatttt-3' (SEQ ID NO: 24) |
| P9_A492L_F | 5'-catccgcctctgaaattctgctagcggcccttcaggattac-3' (SEQ ID NO: 25) |
| P9_A492L_R | 5'-gtaatcctgaagggccgctagcagaatttcagaggcggatg-3' (SEQ ID NO: 26) |
| P9_A492V_F | 5'-cctctgaaattctggttgcggcccttcagga-3' (SEQ ID NO: 27) |
| P9_A492V_R | 5'-tcctgaagggccgcaaccagaatttcagagg-3' (SEQ ID NO: 28) |
| P9_L495I_F | 5'-ttctggctgcggccattcaggattacggc-3' (SEQ ID NO: 29) |
| P9_L495I_R | 5'-gccgtaatcctgaatggccgcagccagaa-3' (SEQ ID NO: 30) |
| P9_A501V_F | 5'-gattacggccgcgtcgtgattgtgggg-3' (SEQ ID NO: 31) |
| P9_A501V_R | 5'-ccccacaatcacgacgcggccgtaatc-3' (SEQ ID NO: 32) |
| P9_A501I_F | 5'-caggattacggccgcatcgtgattgtggggga-3' (SEQ ID NO: 33) |
| P9_A501I_R | 5'-tcccccacaatcacgatgcggccgtaatcctg-3' (SEQ ID NO: 34) |
| P9_G733A_F | 5'-ggaactccgcgaagccatcaacatcgtcc-3' (SEQ ID NO: 35) |
| P9_G733A_R | 5'-ggacgatgttgatggcttcgcggagttcc-3' (SEQ ID NO: 36) |
| P9_G733V_F | 5'-cggaactccgcgaagtcatcaacatcgtcca-3' (SEQ ID NO: 37) |
| P9_G733V_R | 5'-tggacgatgttgatgacttcgcggagttccg-3' (SEQ ID NO: 38) |
| P9_G369A_F | 5'-gaaggatgaacttgccaaagctgaaatcattgaacttacc-3' (SEQ ID NO: 39) |
| P9_G369A_R | 5'-ggtaagttcaatgatttcagctttggcaagttcatccttc-3' (SEQ ID NO: 40) |
| P9 K368Q F | 5'-cctctgaaggatgaacttgcccagggtgaaatcattgaacttacc-3' (SEQ ID NO: 41) |
| P9 K368Q R | 5'-ggtaagttcaatgatttcaccctgggcaagttcatccttcagagg-3' (SEQ ID NO: 42) |
| P9_C589S_F | 5'-atgaccagattacgcccagcaccaactacaaaaag-3' (SEQ ID NO: 43) |
| P9_C589S_R | 5'-ctttttgtagttggtgctgggcgtaatctggtcat-3' (SEQ ID NO: 44) |
| P9_C589T_F | 5'-ctatgaccagattacgcccaccaccaactacaaaaaggact-3' (SEQ ID NO: 45) |
| P9_C589T_R | 5'-agtcctttttgtagttggtggtgggcgtaatctggtcatag-3' (SEQ ID NO: 46) |
| P9_C34A_F | 5'-ccgccattacttccgccgcctctgcggcta-3' (SEQ ID NO: 47) |
| P9_C34A_R | 5'-tagccgcagaggcggcggaagtaatggcgg-3' (SEQ ID NO: 48) |
| P9_V78L_F | 5'-gaaacctacctgcgcaagttagaccccaacaaaatat-3' (SEQ ID NO: 49) |
| P9_V78L_R | 5'-atattttgttggggtctaacttgcgcaggtaggtttc-3' (SEQ ID NO: 50) |
| P9_A251P_F | 5'-gcatacggattacatgggtccgcgccaagt-3' (SEQ ID NO: 51) |
| P9_A251P _R | 5'-acttggcgcggacccatgtaatccgtatgc-3' (SEQ ID NO: 52) |
| P9_D307A_F | 5'-gcatcgttgccattgcctccgacaactctgg-3' (SEQ ID NO: 53) |
| P9_D307A_R | 5'-ccagagttgtcggaggcaatggcaacgatgc-3' (SEQ ID NO: 54) |
| P9_K324D_F | 5'-ctgttcatgaagctggacgatgtggtggatatgatccgc-3' (SEQ ID NO: 55) |
| P9_K324D_R | 5'-gcggatcatatccaccacatcgtccagcttcatgaacag-3' (SEQ ID NO: 56) |
| P9_K324N_F | 5'-cctgttcatgaagctggacaatgtggtggatatg-3' (SEQ ID NO: 57) |
| P9_K324N_R | 5'-catatccaccacattgtccagcttcatgaacagg-3' (SEQ ID NO: 58) |
| P9_M213Q_F | 5'-cccgcggaggaaaaactgcttcagcgttatgaacgc-3' (SEQ ID NO: 59) |
| P9_M213Q_R | 5'-gcgttcataacgctgaagcagtttttcctccgcggg-3' (SEQ ID NO: 60) |
| P9_R402S_F | 5'-ggcggagaccgcagctgtgccaagg-3' (SEQ ID NO: 61) |
| P9_R402S_R | 5'-ccttggcacagctgcggtctccgcc-3' (SEQ ID NO: 62) |
| P9_M414F_F | 5'-Gtcaaaaaaatcctggaacggttcaacaaggaaaatgtggatggc-3' (SEQ ID NO: 63) |
| P9_M414F_R | 5'-gtcaaaaaaatcctggaacggttcaacaaggaaaatgtggatggc-3' (SEQ ID NO: 64) |
| P9_M414I_F | 5'-caaaaaaatcctggaacggataaacaaggaaaatgtggatgg-3' (SEQ ID NO: 65) |
| P9_M414I_R | 5'-ccatccacattttccttgtttatccgttccaggatttttttg-3'(SEQ ID NO: 66) |
| P9_R438V_F | 5'-ccctggaggaagtggtcctgatgacgggct-3' (SEQ ID NO: 67) |
| P9 R438V R | 5'-agcccgtcatcaggaccacttcctccaggg-3' (SEQ ID NO: 68) |
| P9_A465D_F | 5'-cgtggatatcaaatccgaccacaaccgccagaaac-3' (SEQ ID NO: 69) |
| P9_A465D_R | 5'-gtttctggcggttgtggtcggatttgatatccacg-3' (SEQ ID NO: 70) |
| P9_N467D_F | 5'-atatcaaatccgcccacgaccgccagaaactcttc-3' (SEQ ID NO: 71) |
| P9_N467D_R | 5'-gaagagtttctggcggtcgtgggcggatttgatat-3' (SEQ ID NO: 72) |
| P9_A534F_F | 5'-ttcttcgcggccagagaccgtttcggcctgctgaa-3' (SEQ ID NO: 73) |
| P9_A534F_R | 5'-ttcagcaggccgaaacggtctctggccgcgaagaa-3' (SEQ ID NO: 74) |
| P9_Y583F_F | 5'-gactacgcgatgccctttgaccagattacgc-3' (SEQ ID NO: 75) |
| P9_Y583F_R | 5'-gcgtaatctggtcaaagggcatcgcgtagtc-3' (SEQ ID NO: 76) |
| P9_A231F_F | 5'-acggatctggaagacgtattcgaaacactgctcagcgcc-3' (SEQ ID NO: 77) |
| P9_A231F _R | 5'-ggcgctgagcagtgtttcgaatacgtcttccagatccgt-3' (SEQ ID NO: 78) |
| P9_T263L_F | 5'-gtttcaaaatctccatgggtctagaactcaccggcatcggcgc-3' (SEQ ID NO: 79) |
| P9_T263L_R | 5'-Gcgccgatgccggtgagttctagacccatggagattttgaaac-3' (SEQ ID NO: 80) |
| P9_C403T_F | 5'-cggagaccgccgcactgccaaggatgtc-3' (SEQ ID NO: 81) |
| P9_C403T_R | 5'-gacatccttggcagtgcggcggtctccg-3' (SEQ ID NO: 82) |
| P9_T445I_F | 5'-tgatgacgggcttctttatcggaaacggccc-3' (SEQ ID NO: 83) |
| P9_T445I_R | 5'-Gggccgtttccgataaagaagcccgtcatca-3' (SEQ ID NO: 84) |
| P9_C589A_F | 5'-ctatgaccagattacgcccgccaccaactacaaaaaggact-3' (SEQ ID NO: 85) |
| P9_C589A_R | 5'-agtcctttttgtagttggtggcgggcgtaatctggtcatag-3' (SEQ ID NO: 86) |
| P9_C403A_F | 5'-cggagaccgccgcgctgccaaggatgtc-3' (SEQ ID NO: 87) |
| P9_C403A_R | 5'-gacatccttggcagcgcggcggtctccg-3' (SEQ ID NO: 88) |
| P9_K46A_F | 5'-cttcaaccaggtgggcgcgcaaatgtccctgctg-3' (SEQ ID NO: 89) |
| P9_K46A_R | 5'-cagcagggacatttgcgcgcccacctggttgaag-3' (SEQ ID NO: 90) |
| P9_R59A/K60A_F | 5'-gctccagaatttccacttctccgccgcagaattcagcgatgaactatcc-3' (SEQ ID NO: 91) |
| P9_R59A/K60A_R | 5'-ggatagttcatcgctgaattctgcggcggagaagtggaaattctggagc-3' (SEQ ID NO: 92) |
| P9_K82A_F | 5'-gcgcaaggtagaccccaacgcaatattcttcacccagcag-3' (SEQ ID NO: 93) |
| P9_K82A_R | 5'-ctgctgggtgaagaatattgcgttggggtctaccttgcgc-3' (SEQ ID NO: 94) |
| P9_K94A/R95A/K96 A_F | 5'-ccagcaggacgtagacgccctcgcagcagcatacggtaaggagctggacgac-3' (SEQ ID NO: 95) |
| P9_K94A/R95A/K96 A_R | 5'- gtcgtccagctccttaccgtatgctgctgcgagggcgtctacgtcctgctgg-3' (SEQ ID NO: 96) |
| P9_K368A_F | 5'-cctctgaaggatgaacttgccgcaggtgaaatcattgaacttac-3' (SEQ ID NO: 97) |
| P9_K368A_R | 5'-gtaagttcaatgatttcacctgcggcaagttcatccttcagagg-3' (SEQ ID NO: 98) |
| P9_K482A_F | 5'-gccccattgtggtgctcatcaatgcactcagcgcatc-3' (SEQ ID NO: 99) |
| P9_K482A_R | 5'-gatgcgctgagtgcattgatgagcaccacaatggggc-3' (SEQ ID NO: 100) |
| P9_R500A_F | 5'-cttcaggattacggcgccgccgtgattgtggg-3' (SEQ ID NO: 101) |
| P9_R500A_R | 5'-cccacaatcacggcggcgccgtaatcctgaag-3' (SEQ ID NO: 102) |
| P9_K512A_F | 5'-gaatccaccttcggggcggggtctgtgcagca-3' (SEQ ID NO: 103) |
| P9_K512A_R | 5'-tgctgcacagaccccgccccgaaggtggattc-3' (SEQ ID NO: 104) |
| P9_R531A_F | 5'-tttcttcgcggccgcagaccgtgcgggc-3' (SEQ ID NO: 105) |
| P9_R531A_R | 5'-gcccgcacggtctgcggccgcgaagaaa-3' (SEQ ID NO: 106) |
| P9_C34S_F | 5'-cgccattacttccagcgcctctgcgca-3'(SEQ ID NO: 107) |
| P9_C34S_R | 5'-tgcgcagaggcgctggaagtaatggcg-3'(SEQ ID NO: 108) |
| P9_S36H/A37H_F | 5'-cgccattacttcctgcgcccatcatcaccaccaccaccaccacg-3' (SEQ ID NO: 109) |
| P9_S36H/A37H_R | 5'-cgtggtggtggtggtggtgatgatgggcgcaggaagtaatggcg-3'(SEQ ID NO: 110) |
| P9_S486A_F | 5'-gctcatcaataaactcagcgcagccgcctctgaaatt-3' (SEQ ID NO: 111) |
| P9_S486A_R | 5'-aatttcagaggcggctgcgctgagtttattgatgagc-3' (SEQ ID NO: 112) |

As a result of confirming the mutation, it was confirmed that plasmids for 63 additional P9-02-based variants described in Table 8 were constructed.

**[Table 8]**

| | | | | |
|---|---|---|---|---|
| C34A | A251P | R402S | V423I | A501V |
| C34S | T263I | R402S/Y583F | R438V | K512A |
| S36H/A37H | T263L | C403A | R438V/G446R | R531A |
| G45A | T263V | C403A/C589A | T445I | A534F |
| G45L | D307A | C403A/C589A/S36H/A37H | A465D | C589A |
| G45V | K324D | C403A/C589A/K46A | N467D | C589A/C34S |
| K46A | K324D/A534V | C403A/C589A/K82A | K482A | C589A/S36H/A37H |
| R59A/K60A | K324N | C403A/C589A/K94A/R95A/K96A | S486A | C589S |
| V78L | K368A | C403A/C589A/K368A | A492L | C589T |
| K82A | K368A/K46A | C403T | A492V | G733A |
| K94A/R95A/K96A | K368A/K82A | M414F | A495I | G733V |
| M213Q | K368A/K94A/R95A/K96A | M414! | R500A | |
| A231F | G369A | M414I/K555R | A501I | |

In the table above, K324D/A534V, M414I/K555R, and R438V/G446R were obtained due to PCR error. Also, among multiple variants, K368A/K46A, K368A/K82A, K368A/K94A/R95A/K96A, C403A/C589A, C403A/C589A/S36H/A37H, C403A/C589A/K46A, C403A/C589A/K82A, C403A/C589A/C K94A/R95A/K96A, C403A/C589A/K368A, C589A/C34S, and C589A/S36H/A37H were constructed by sequential mutagenesis using primers for construction of each corresponding single variant.

A plasmid suitable for expression of each variant was transformed into *E. coli* in the same manner as in Example 2 to express each variant, and each variant was purified in the same manner as in Example 2.

### Example 5: Confirmation of GLP-1 inducibility of additional P9-02((10-37)-His-(38-748))-based variants

The GLP-1 inducibility of the variants was measured in the same manner as in Example 3-1, and the increase or decrease of GLP-1 compared to P9-02 of each of the additional P9-02-based variants is shown in Table 9 below.

**[Table 9]**

| | **P9 variants** | **1st Experiment** | | **2nd Experiment** | |
|---|---|---|---|---|---|
| | | fold | p-value | fold | p-value |
| **1** | P9-02((10-37)-His-(38-748)) | 1.00 | | | |
| **2** | C34A | 0.33 | *** | | |
| **3** | C34S | 0.27 | *** | 0.27 | *** |
| **4** | S36H/A37H | 1.05 | - | 1.03 | - |
| **5** | G45A | 1.32 | ** | | |
| **6** | G45L | 0.01 | *** | | |
| **7** | G45V | 0.01 | *** | | |
| **8** | K46A | 0.69 | ** | | |
| **9** | R59A/K60A | 0.39 | *** | | |
| **10** | V78L | 0.73 | - | | |
| **11** | K82A | 0.76 | ** | | |
| **12** | K94A/R95A/K96A | 1.01 | - | 1.03 | - |
| **13** | M213Q | 1.00 | - | | |
| **14** | A231F | 0.00 | *** | | |
| **15** | A251P | 0.48 | *** | | |
| **16** | T263I | 1.41 | ** | | |
| **17** | T263L | 0.07 | - | | |
| **18** | T263V | 1.24 | * | | |
| **19** | D307A | 0.97 | - | | |
| **20** | K324D | 0.35 | - | | |
| **21** | K324D/A534V | 0.01 | *** | | |
| **22** | K324N | 0.74 | *** | | |
| **23** | K368A | 1.28 | *** | | |
| **24** | K368A/K46A | 0.75 | *** | | |
| **25** | K368A/K82A | 0.56 | *** | | |
| **26** | K368A/K94A/R95A/K96A | 1.58 | *** | | |
| **27** | G369A | 0.53 | ** | | |
| **28** | R402S | 0.34 | *** | | |
| **29** | R402S/Y583F | 0.49 | *** | | |
| **30** | C403A | 1.24 | ** | 1.61 | *** |
| **31** | C403A/C589A | 1.62 | *** | 1.50 | * |
| **32** | C403A/C589A/S36H/A37H | 1.11 | * | 1.53 | * |
| **33** | C403A/C589A/K46A | 0.93 | - | 1.06 | - |
| **34** | C403A/C589A/K82A | 1.15 | ** | 1.47 | ** |
| **35** | C403A/C589A/K94A/ | 1.40 | * | | |
| | R95A/K96A | | | | |
| **36** | C403A/C589A/K368A | 1.06 | - | 1.45 | * |
| **37** | C403T | 0.07 | *** | | |
| **38** | M414F | 0.84 | - | | |
| **39** | M414I | 0.23 | *** | | |
| **40** | M414I/K555R | 0.71 | * | | |
| **41** | V423I | 0.44 | *** | | |
| **42** | R438V | 0.31 | *** | | |
| **43** | R438V/G446R | 0.32 | *** | | |
| **44** | T445I | 0.76 | - | 0.97 | - |
| **45** | A465D | 0.07 | *** | | |
| **46** | N467D | 0.47 | *** | | |
| **47** | K482A | 0.06 | *** | | |
| **48** | S486A | 0.91 | - | 0.95 | - |
| **49** | A492L | 1.02 | - | | |
| **50** | A492V | 0.16 | *** | | |
| **51** | A495I | 0.45 | *** | | |
| **52** | R500A | 0.38 | *** | | |
| **53** | A501I | 0.01 | *** | | |
| **54** | A501V | 0.00 | *** | | |
| **55** | K512A | 0.00 | *** | | |
| **56** | R531A | 0.41 | *** | | |
| **57** | A534F | 0.21 | *** | | |
| **58** | C589A | 2.59 | * | 4.44 | *** |
| **59** | C589A/C34S | 0.51 | ** | | |
| **60** | C589A/S36H/A37H | 1.44 | ** | | |
| **61** | C589S | 1.78 | *** | | |
| **62** | C589T | 1.49 | *** | | |
| **63** | G733A | 1.24 | ** | | |
| **64** | G733V | 1.29 | *** | | |

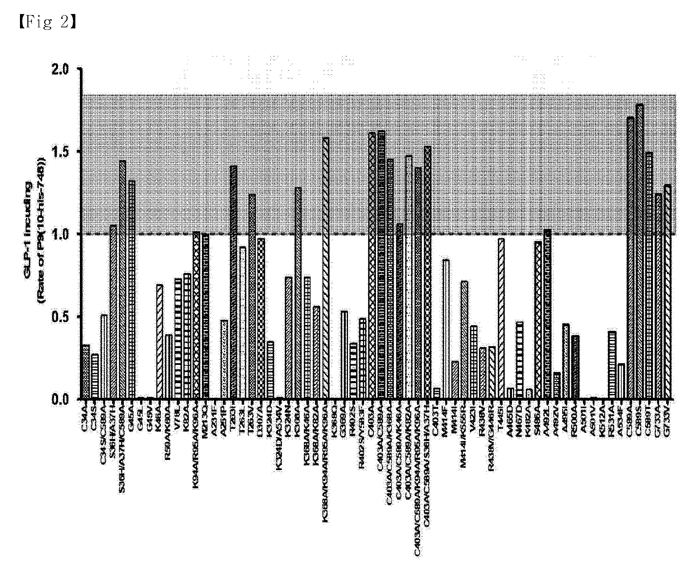

As shown in Table 9 and Figure 2 above, among the additional P9-02-based variants, the variants further including mutations of G733A, G45A, T263I, T263V, K368A, K368A/K94A/R95A/K96A, C403A, C403A/C589A, C403A/C589A/S36H/A37H, C403A/C589A/K82A, C403A/C589A/K94A/R95A/K96A, C403A/C589A/K368A, C589A, C589A/S36H/A37H, C589S, C589T and G733V has significantly improved GLP-1 inducibility than P9-02.

### Example 6: Confirmation of temperature stability of additional P9-02((10-37)-His-(38-748))-based variants

In order to confirm the temperature stability of each of variants prepared in Example 4, the Tm of each protein was measured using the Protein Thermal Shift^{™} Dye Kit (ThermoFisher Scientific, Cat#4461146) according to the manufacturer's protocol.

In order to initiate the melting reaction of the protein, Protein Thermal Shift^{™} dye (1000X) was prepared by diluting 8X, and the reaction plate was placed on ice. 5 µL Protein Thermal Shift^{™} buffer and 12.5 µL protein sample were added to the reaction plate. 2.5 µL of the diluted 8X Protein Thermal Shift Dye was added and mixed by pipetting up and down 10 times. The plate was sealed with MicroAmp^{®} optical adhesive film and spun at 1000 rpm for 1 min and then the plate was placed on ice.

For analysis, the plates were loaded and real-time PCR machine was run with the following settings: reaction volume per well: 20 µL, mode ramp mode: continuous, profile thermal profile (operation 1, temperature 14 °C, 2 min, 1.6 °C/s; operation 2, temperature rise at 0.05 °C/s to temperature 99 °C), excitation filter: x4 (580 nm), emission filter: m4 (623 nm) optical filter.

As a result of the experiment, the Tm of P9-01(10-748-His) and P9-02((10-37)-His-(38-748)) were similarly measured at 47.6 °C and 47.2 °C, respectively.

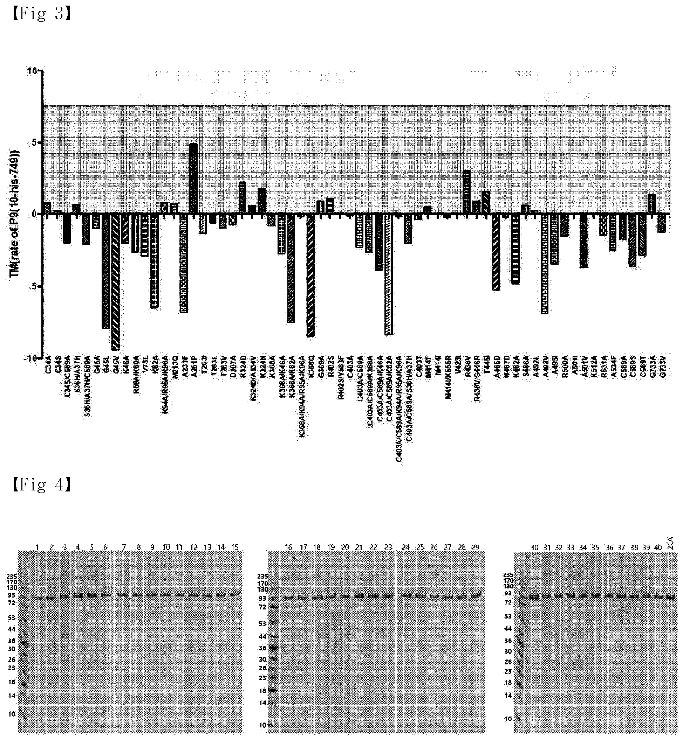

Meanwhile, it was observed that Tm was improved in P9 variants including mutations of S36H/A37H, K94A/R95A/K96A, M213Q, M414F, T445I, S486A and A492L among additional P9-02-based variants compared to P9-02 (FIG. 3).

The increase in Tm relative to P9-02 of each of the additional P9-02-based variants is shown in Table 10 below.

**[Table 10]**

| | **P9 variants** | **ΔTm(°C)** | | **P9 variants** | **ΔTm(°C)** |
|---|---|---|---|---|---|
| **1** | P9-02((10-37)-His-(38-748)) | 0 | **33** | C403A/C589A/K46A | -3.9 |
| **2** | C34A | 0.8 | **34** | C403A/C589A/K82A | -8.4 |
| **3** | C34S | 0.3 | **35** | C403A/C589A/K94A/R95 A/K96A | N/A |
| **4** | S36H/A37H | 0.7 | **36** | C403A/C589A/K368A | -2.6 |
| **5** | G45A | -1.0 | **37** | C403T | -0.3 |
| **6** | G45L | -7.9 | **38** | M414F | 0.5 |
| **7** | G45V | -9.4 | **39** | M414I | 0.0 |
| **8** | K46A | -2.0 | **40** | M414I/K555R | -0.2 |
| **9** | R59A/K60A | -2.6 | **41** | V423I | 0.0 |
| **10** | V78L | -2.9 | **42** | R438V | 3.0 |
| **11** | K82A | -6.5 | **43** | R438V/G446R | 0.9 |
| **12** | K94A/R95A/K96A | 0.8 | **44** | T445I | 1.6 |
| **13** | M213Q | 0.7 | **45** | A465D | -5.3 |
| **14** | A231F | -6.8 | **46** | N467D | -0.2 |
| **15** | A251P | 4.9 | **47** | K482A | -4.8 |
| **16** | T263I | -1.3 | **48** | S486A | 0.6 |
| **17** | T263L | -0.6 | **49** | A492L | 0.2 |
| **18** | T263V | -0.9 | **50** | A492V | -6.9 |
| **19** | D307A | -0.7 | **51** | A495I | -3.5 |
| **20** | K324D | 2.2 | **52** | R500A | -1.5 |
| **21** | K324D/A534V | 0.6 | **53** | A501I | N/D |
| **22** | K324N | 1.8 | **54** | A501V | -3.7 |
| **23** | K368A | -0.8 | **55** | K512A | 0.1 |
| **24** | K368A/K46A | -2.7 | **56** | R531A | -1.5 |
| **25** | K368A/K82A | -7.5 | **57** | A534F | -2.6 |
| **26** | K368A/K94A/R95A/K96A | -0.2 | **58** | C589A | -1.7 |
| **27** | G369A | 0.9 | **59** | C589A/C34S | -2.0 |
| **28** | R402S | 1.1 | **60** | C589A/S36H/A37H | -2.0 |
| **29** | R402S/Y583F | 0.1 | **61** | C589S | -3.6 |
| **30** | C403A | -0.1 | **62** | C589T | -2.9 |
| **31** | C403A/C589A | -2.3 | **63** | G733A | 1.4 |
| **32** | C403A/C589A/S36H/A37H | -2.0 | **64** | G733V | -1.2 |

Through the above experimental results, it was found that the variants further including mutations of G733A, S36H/A37H, K94A/R95A/K96A, M213Q, M414F, T445I, S486A, and A492L in P9-02 exhibit improved temperature stability compared to P9-02. In particular, it was confirmed that both GLP-1 inducibility and temperature stability were improved in the P9 protein variant further including the G733A mutation in P9-02.

### Example 7: Preparation of additional P9-2CA(P9-02((10-37)-His-(38-748)) C403A/C589A)-based variants

Using the pET26b::P9-02((10-37)-His-(38-748)) C403A/C589A constructed in Example 4 as a template, additional variants were constructed using the same method as in Example 4.

Table 11 below shows the primer sequences for site-directed mutagenesis of SEQ ID NOs: 114 to 193 (in the 5'->3' direction).

**[Table 11]**

| | Aditional mutation | Forward | Reverse |
|---|---|---|---|
| 1 | Q43C | gcggctacggacttcaactgcgtgggcaagcaaat gtcc (SEQ ID NO: 114) | ggacatttgcttgcccacgcagttgaagtccgtagcc gc (SEQ ID NO: 115) |
| 2 | R59C | cagaatttccacttctcctgcaaagaattcagcgatg (SEQ ID NO: 116) | catcgctgaattctttgcaggagaagtggaaattctg (SEQ ID NO: 117) |
| 3 | R95C | gacgtagacgccctcaaatgcaaatacggtaagga gctg (SEQ ID NO: 118) gccctcaaaagaaaatacggttgcgagctggacg actaccttatg (SEQ ID NO: 120) | cagctccttaccgtatttgcatttgagggcgtctacgtc (SEQ ID NO: 119) |
| 4 | K99C | gccctcaaaagaaaatacggttgcgagctggacg actaccttatg (SEQ ID NO: 120) | cataaggtagtcgtccagctcgcaaccgtattttcttttg agggc (SEQ ID NO: 121) |
| 5 | S107C | ctggacgactaccttatgtgcggccagatg (SEQ ID NO: 122) | catctggccgcacataaggtagtcgtccag (SEQ ID NO: 123) |
| 6 | A119C | cccaggccatgcactgcctttaccgccagc (SEQ ID NO: 124) | gctggcggtaaaggcagtgcatggcctggg (SEQ ID NO: 125) |
| 7 | K138C | cctatgcgcgggatttgctgaaatgcggaggcttca cc (SEQ ID NO: 126) | ggtgaagcctccgcatttcagcaaatcccgcgcatag g (SEQ ID NO: 127) |
| 8 | T142C | gctgaaaaagggaggcttctgctttgacaaagaca agtct (SEQ ID NO: 128) | agacttgtctttgtcaaagcagaagcctccctttttcag c (SEQ ID NO: 129) |
| 9 | A158C | cgccgcaaaacagcctgctggcccaaggatgag (SEQ ID NO: 130) | ctcatccttgggccagcaggctgttttgcggcg (SEQ ID NO: 131) |
| 10 | K161C | acagccgcgtggccctgcgatgaggcggaaatg (SEQ ID NO: 132) | catttccgcctcatcgcagggccacgcggctgt (SEQ ID NO: 133) |
| 11 | A164C | cgtggcccaaggatgagtgcgaaatgcagcaggt ctg (SEQ ID NO: 134) | cagacctgctgcatttcgcactcatccttgggccacg (SEQ ID NO: 135) |
| 12 | A189C | cctgcgccgtgaaaccgtatgccgcctggcca (SEQ ID NO: 136) | tggccaggcggcatacggtttcacggcgcagg (SEQ ID NO: 137) |
| 13 | K193C | gtagcgcgcctggcctgcgaacagaacaagccc (SEQ ID NO: 138) | gggcttgttctgttcgcaggccaggcgcgctac (SEQ ID NO: 139) |
| 14 | N203C | ccgatcccctggcctgtgaaaaacccgcgg (SEQ ID NO: 140) | ccgcgggtttttcacaggccaggggatcgg (SEQ ID NO: 141) |
| 15 | R220C | gcgttatgaacgcattcagtgcaatattcaggaaac gg (SEQ ID NO: 142) | ccgtttcctgaatattgcactgaatgcgttcataacgc (SEQ ID NO: 143) |
| 16 | K258C | gcgccaagtggaccgtttctgcatctccatgggtac ggaac (SEQ ID NO: 144) | gttccgtacccatggagatgcagaaacggtccacttg gcgc (SEQ ID NO: 145) |
| 17 | S311C | gccattgactccgacaactgtggagaaatggtg (SEQ ID NO: 146) | caccatttctccacagttgtcggagtcaatggc (SEQ ID NO: 147) |
| 18 | A332C | gtggtggatatgatccgcggatgcgaaaataccca ga (SEQ ID NO: 148) | tctgggtattttcgcatccgcggatcatatccaccac (SEQ ID NO: 149) |
| 19 | S358C | acgctgacccgctgcaaggtacctctg (SEQ ID NO: 150) | cagaggtaccttgcagcgggtcagcgt (SEQ ID NO: 151) |
| 20 | K363C | ccgctccaaggtacctctgtgcgatgaacttgccaa aggtg (SEQ ID NO: 152) | caccctttggcaagttcatcgcacagaggtaccttgga gcgg (SEQ ID NO: 153) |
| 21 | E379C | aacttaccggagctccgtgcggcaggaaccgcatt gg (SEQ ID NO: 154) | ccaatgcggttcctgccgcacggagctccggtaagtt (SEQ ID NO: 155) |
| 22 | K409C | gccaaggatgtcaaatgcatcctggaacggatgaa caa (SEQ ID NO: 156) | ttgttcatccgttccaggatgcatttgacatccttggc (SEQ ID NO: 157) |
| 23 | K416C | aaaatcctggaacggatgaactgcgaaaatgtgga tggcctggta (SEQ ID NO: 158) | taccaggccatccacattttcgcagttcatccgttccag gatttt (SEQ ID NO: 159) |
| 24 | S464C | gcggcaacgtggatatcaaatgcgcccacaacc (SEQ ID NO: 160) | ggttgtgggcgcatttgatatccacgttgccgc (SEQ ID NO: 161) |
| 25 | R468C | tcaaatccgcccacaactgccagaaactcttcaat (SEQ ID NO: 162) | attgaagagtttctggcagttgtgggcggatttga (SEQ ID NO: 163) |
| 26 | Q523C | cagcctgtggacatcggctgctacctgcctttcttcg cg (SEQ ID NO: 164) | cgcgaagaaaggcaggtagcagccgatgtccacag gctg (SEQ ID NO: 165) |
| 27 | A530C | tgcctttcttcgcgtgcagagaccgtgcgg (SEQ ID NO: 166) | ccgcacggtctctgcacgcgaagaaaggca (SEQ ID NO: 167) |
| 28 | Q562C | caaaggcgtggaaagcgatatctgccttcccaccg ctacg (SEQ ID NO: 168) | cgtagcggtgggaaggcagatatcgctttccacgcct ttg (SEQ ID NO: 169) |
| 29 | D575C | ggcattcgaactgggagaatgcattctggactacg cgatg (SEQ ID NO: 170) | catcgcgtagtccagaatgcattctcccagttcgaatg cc (SEQ ID NO: 171) |
| 30 | Q585C | tacgcgatgccctatgactgcattacgcccgccacc aac (SEQ ID NO: 172) | gttggtggcgggcgtaatgcagtcatagggcatcgc gta (SEQ ID NO: 173) |
| 31 | T590C | gaccagattacgcccgcctgcaactacaaaaagg actc (SEQ ID NO: 174) | gagtcctttttgtagttgcaggcgggcgtaatctggtc (SEQ ID NO: 175) |
| 32 | S596C | ccaactacaaaaaggactgctccatcgcggccat (SEQ ID NO: 176) | atggccgcgatggagcagtcctttttgtagttgg (SEQ ID NO: 177) |
| 33 | A610C | gtgctgaaagatgccagctgcaagcgcgtggaaa aagac (SEQ ID NO: 178) | gtctttttccacgcgcttgcagctggcatctttcagcac (SEQ ID NO: 179) |
| 34 | A627C | gattgccagggaagatatctgcatgatgaaacagc gcatc (SEQ ID NO: 180) | gatgcgctgtttcatcatgcagatatcttccctggcaat c (SEQ ID NO: 181) |
| 35 | K634C | gccatgatgaaacagcgcatctgcgacaacaagct ttccctgaac (SEQ ID NO: 182) | gttcagggaaagcttgttgtcgcagatgcgctgtttcat catggc (SEQ ID NO: 183) |
| 36 | A651C | ggaacaggaaaactcctgcctggaagaacgccgc (SEQ ID NO: 184) | gcggcgttcttccaggcaggagttttcctgttcc (SEQ ID NO: 185) |
| 37 | S658C | ggaagaacgccgcaaatgcatcaacaaggaacgt (SEQ ID NO: 186) | acgttccttgttgatgcatttgcggcgttcttcc (SEQ ID NO: 187) |
| 38 | K700C | gcccctggcggatccggaatgcgacaatgaacaa ttcatgca (SEQ ID NO: 188) | tgcatgaattgttcattgtcgcattccggatccgccagg ggc (SEQ ID NO: 189) |
| 39 | E710C | caattcatgcacctggcgtgcgaccccacggcaga actg (SEQ ID NO: 190) | cagttctgccgtggggtcgcacgccaggtgcatgaat tg (SEQ ID NO: 191) |
| 40 | S745C | caggatatgctgaagctggaatgctccggaaaatg (SEQ ID NO: 192) | cattttccggagcattccagcttcagcatatcctg (SEQ ID NO: 193) |

As a result, it was confirmed that plasmids for 40 additional P9-2CA-based variants described in Table 12 were constructed.

**[Table 12]**

| | | | |
|---|---|---|---|
| Q43C/C403A/C589A | A164C/C403A/C589A | E379C/C403A/C589A | C403A/C589A/T590C |
| R59C/C403A/C589A | A189C/C403A/C589A | C403A/K409C/C589A | C403A/C589A/S596C |
| R95C/C403A/C589A | K193C/C403A/C589A | C403A/K416C/C589A | C403A/C589A/A610C |
| K99C/C403A/C589A | N203C/C403A/C589A | C403A/S464C/C589A | C403A/C589A/A627C |
| S107C/C403A/C589A | R220C/C403A/C589A | C403A/R468C/C589A | C403A/C589A/K634C |
| A119C/C403A/C589A | K258C/C403A/C589A | C403A/Q523C/C589A | C403A/C589A/A651C |
| K138C/C403A/C589A | S311C/C403A/C589A | C403A/A530C/C589A | C403A/C589A/S658C |
| T142C/C403A/C589A | A332C/C403A/C589A | C403A/Q562C/C589A | C403A/C589A/K700C |
| A158C/C403A/C589A | S358C/C403A/C589A | C403A/D575C/C589A | C403A/C589A/E710C |
| K161C/C403A/C589A | K363C/C403A/C589A | C403A/Q585C/C589A | C403A/C589A/S745C |

*E. coli* was transformed with a plasmid suitable for expression of each variant as follows to express and purify each variant. Colonies were inoculated into 10 mL of TB medium supplemented with 34 µg/ml chloramphenicol and 50 µg/ml kanamycin to prepared a fresh culture of *E. coli* BL21 (DE3) carrying a plasmid for expression of additional P9-2CA-based variants. 10 ml of the cultures were inoculated into 500 mL of TB medium supplemented with 0.5% (w/v) glycerol, 0.05% (w/v) glucose, 0.4% (w/v) lactose, 34 µg/ml chloramphenicol and 50 µg/ml kanamycin. Cells were incubated for 16-18 hours overnight at 25 °C with shaking at 200 rpm until the OD₆₀₀ value reached 4.0. Thereafter, the cells were harvested by centrifugation (6000 g, 20 min) and the cell pellet was frozen at <-60 °C until use.

Cells were resuspended in cooled 50 mL lysis buffer containing 1X PBS (20X PBS dilution, BIOSESANG) and 2 mM 2-mercaptoethanol, and sonicated in the same manner as in Example 2 (amplification 40%). Thereafter, the sample was cooled for 5 minutes, the sonication operation was repeated. Then, cell debris was removed by centrifugation at 20,000 g for 20 minutes at 4 °C.

41 kinds of P9 variants (P9-2CA variants, and 40 kinds of additional P9-2CA-based variants) were purified via Ni-NTA and anion-exchange chromatography.

First, in order to perform Ni-NTA chromatography, an open column containing 2 ml Ni-NTA Sepharose resin equilibrated with a lysis buffer was prepared. The prepared column was loaded with cell lysate and rotated for 30 to 60 minutes. The resin was washed with 200 mL wash buffer 1 [20 mM Tris-HCl (pH 8.0) buffer supplemented with 150 mM NaCl, 15 mM imidazole (pH 8.0) and 2 mM 2-mercaptoethanol]. Thereafter, the resin was washed with 50 mL wash buffer 2 [20 mM Tris-HCl (pH 8.0) buffer supplemented with 15 mM imidazole (pH 8.0) and 2 mM 2-mercaptoethanol]. The His-tagged protein was then eluted with 25 mL elution buffer (20 mM Tris-HCl buffer, pH 7.0 supplemented with 200 mM imidazole and 2 mM 2-mercaptoethanol).

Then, the eluted His-tagged protein was collected, and anion exchange chromatography (Hitrap Q HP 5ml) was performed under the following conditions.

Q-A buffer: 20 mM Tris-HCl, 1 mM CaCl₂ and 2 mM TCEP (tris(2-carboxyethyl)phosphine), pH 7.

Q-B buffer: 20 mM Tris-HCl, 1 M NaCl, 1 mM CaCl₂ and 2 mM TCEP (tris(2-carboxyethyl)phosphine), pH 7.

Flow rate: 5 ml/min.

A Hitrap Q HP (5ml) column (manufactured by Cytiva) equilibrated with Q-A buffer was prepared. The His-tagged protein sample was diluted 2-fold with 25 mL Q-A buffer and the sample was loaded with a pump on the equilibrated column. Elution was started by gradually increasing the ionic strength to 0.4 M NaCl (40% Q-B buffer). Fractions containing pure 2CA-based variants were collected. The fractions were analyzed by SDS-PAGE.

The purified protein variant was Cys-specific PEGylated in the following buffer under the presence of the reducing agent TCEP.

Buffer: 20 mM Tris-HCl, 150 mM NaCl and 2 mM TCEP, pH 7.

The concentration of the 2CA-based variants was adjusted to 2.4 µM, and 100x PEGylated reagent containing 5 mM poly(ethylene glycol) methyl ether maleimide (molecular weight 2000) (10 mg/ml) in 100% DMSO was prepared. After reacting the 2.4 µM variant and 50 µM PEGylation reagent at room temperature for 2 hours, the buffer of the PEGylated variant was exchanged with PBS buffer through dialysis.

Unlike other reducing agents such as DTT and 2-mercaptoethanol, TCEP is a reducing agent that does not have a thiol group and therefore does not react with the maleimide group of the PEGylation reagent. Therefore, Cys-specific PEGylation was stably performed through Cys-maleimide reaction while maintaining the cysteine of the P9 variant in a reduced state.

SDS-PAGE results of PEGylated P9-2CA variant and 40 kinds of P9-2CA-based variants are shown in FIG. 4. Each sample was diluted to 0.05 mg/ml and used for cell experiments.

### Example 8: Measurement of GLP-1 inducibility of additional PEGylated P9-2CA(P9-02((10-37)-His-(38-748)) C403A/C589A)-based variants (L-cell)

By measuring the GLP-1 inducibility of 41 kinds of PEGylated variants prepared in Example 7 in the same manner as in Example 3-1, the increase or decrease of GLP-1 are shown in Table 13 below compared to P9-2CA of each of the additional P9-2CA-based variants.

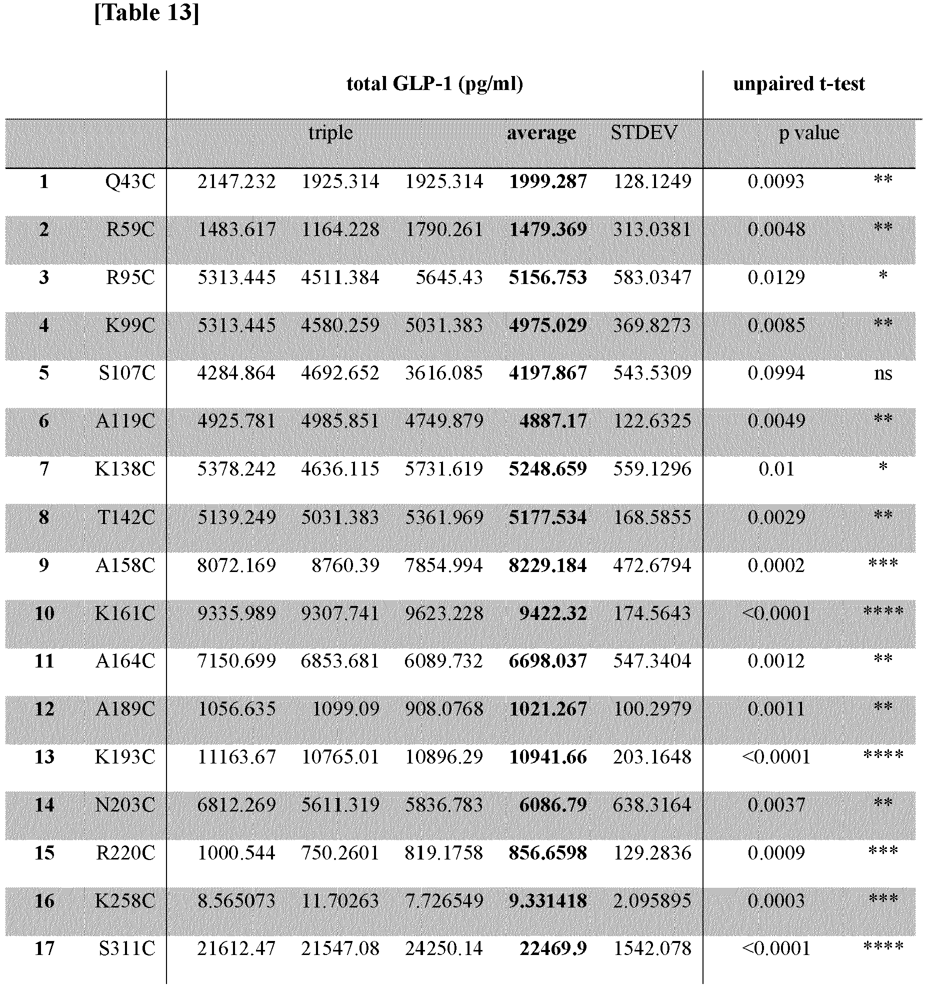

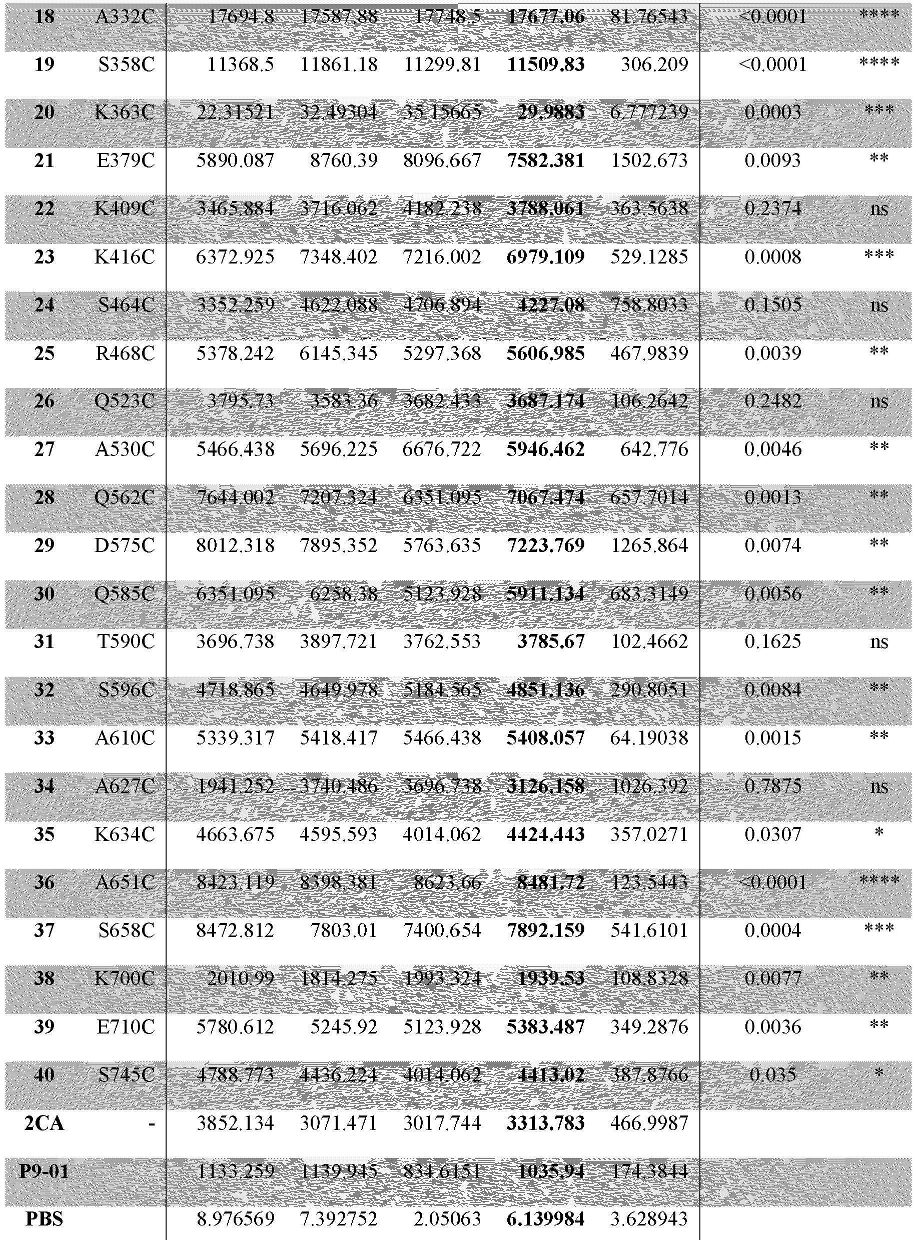

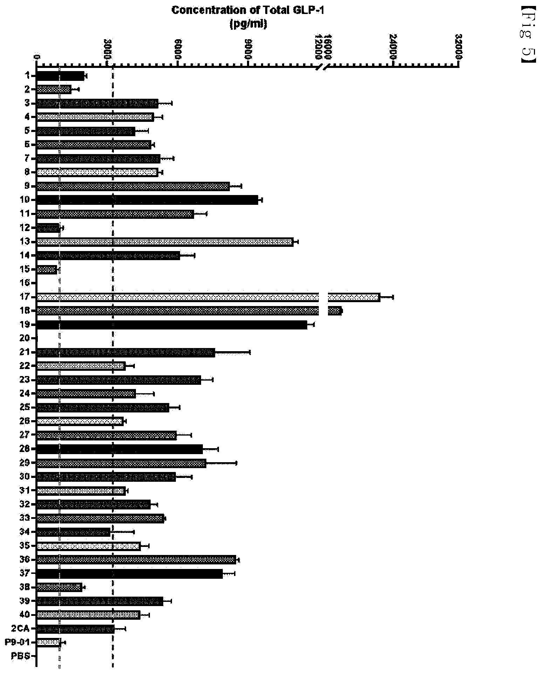

All P9 variants (2CA, No. 1-40) except for P9-01 were Cys-specific PEGylated. Numbers 1 to 40 on the X-axis of the graph in FIG. 5 are numbers arbitrarily assigned to the P9 variant, and are the same as the variants indicated in Table 13. 2CA is a control protein that is a template for each P9 variant (No. 1-40). As shown in Table 13 and FIG. 5, as a result of comparing the GLP-1 inducibility of PEGylated P9, the overall GLP-1 inducibility was increased. When PEGylated to P9 variants (No. 1, 2, 12, 15, 16, 20, and 38 variants) including mutations of Q43C, R59C, A189C, R220C, K258C, K363C, and K700C among additional 2CA-based variants, it was confirmed that the GLP-1 inducibility was decreased compared to 2CA (although the GLP-1 inducibility was increased compared to the wild type). Also, it was confirmed that in P9 variants (No. 4, 6, 7, 8, 9, 10, 11, 13, 14, 17, 18, 19, 21, 23, 25, 27, 28, 29, 30, 32, 33, 36, 37, and 39 variants) including K99C, A119C, K138C, T142C, A158C, K161C, A164C, K193C, N203C, S311C, A332C, S358C, E379C, K416C, R468C, A530C, Q562C, D575C, Q585C, S596C, A610C, A651C, S658C, and E710C among 2CA-based additional variants, the efficacy was increased with a P value <0.01.

### Industrial Applicability

This disclosure relates to a protein variant having improved GLP-1 inducibility and/or thermal stability and enzyme resistance, and a pharmaceutical composition for reducing body weight and/or body fat, suppressing appetite or treating or preventing of metabolic diseases, comprising the same.

## Claims

1. A protein variant comprising the amino acid sequence of SEQ ID NO: 2.

2. A protein variant comprising the amino acid sequence of SEQ ID NO: 4, **characterized by** the amino acid sequence of SEQ ID NO: 2 having hexa-histidine tag at the C-terminus inserted between amino acids 37 and 38.

3. A protein variant comprising at least one or more of the following amino acid substitutions in the amino acid sequence of SEQ ID NO: 4:
| | |
|---|---|
| - C34A, C34S | - C403A, C403T |
| - S36H | - M414F, M414I |
| - A37H | - V423I |
| - G45A, G45L, G45V | - R438V, R438A, R438Q, R438N, R438H |
| - K46A, K46Q, K46N, K46H, K46T | - T445I |
| - R59A, R59Q, R59N, R59H | - G446R |
| - K60A, K60Q, K60N, K60H, K60T | - A465D |
| - V78L | - N467D |
| - K82A, K82Q, K82N, K82H, K82V, K82T | - K482A, K482Q, K482N, K482H, K482T |
| - K94A, K94Q, K94N, K94H, K94T | - S486A |
| - R95A, R95Q, R95N, R95H | - A492L, A492V |
| - K96A, K96Q, K96N, K96H, K96T | - A4951 |
| - M213Q, M213V, M213I, M213A | - R500A |
| - A231F | -A501I, A501V |
| - A251P | - K512A, K512Q, K512N, K512H, K512T |
| - T263I, T263L, T263V | - R531A, R531Q, R531N, R531H |
| - D307A | - A534F, A534V |
| - K324D, K324N, K324A, K324Q, K324N, K324H, K324T | - K555R, K555A, K555Q, K555N, K555H |
| - K368A, K368Q, K368N, K368H, K368T | - Y583F, Y583V, Y583I, Y583A, Y583H |
| - G369A | - C589A, C589S, C589T |
| - R402S, R402A, R402Q, R402N, R402H | - G733A, G733V |

4. The protein variant comprising at least one or more of the following amino acid substitutions in the amino acid sequence of SEQ ID NO: 4:
| | |
|---|---|
| - C34A, C34S | - C403A, C403T |
| - S36H | - M414F, M414I |
| - A37H | - V423I |
| - G45A, G45L, G45V | - R438V |
| - K46A | - T445I |
| - R59A | - G446R |
| - K60A | - A465D |
| - V78L | - N467D |
| - K82A | - K482A |
| - K94A | - S486A |
| - R95A | - A492L, A492V |
| - K96A | - A495I |
| - M213Q | - R500A |
| - A231F | - A501I, A501V |
| - A251P | - K512A |
| - T263I, T263L, T263V | - R531A |
| - D307A | - A534F, A534V |
| - K324D, K324N | - K555R, |
| - K368A | - Y583F, |
| - G369A | - C589A, C589S, C589T |
| - R402S | - G733A, G733V |

5. The protein variant of claim 4, comprising an amino acid substitution in the amino acid sequence of SEQ ID NO:4.

6. The protein variant of claim 4, comprising 2 to 5 amino acid substitutions in the amino acid sequence of SEQ ID NO:4.

7. The protein variant of claim 6, comprising one or more amino acid substitutions of K368A, C403A and C589A.

8. The protein variant of claim 4, comprising any one of the following amino acid substitutions:
| | | | | |
|---|---|---|---|---|
| C34A | A251P | R402S | V423I | A501V |
| C34S | T263I | R402S/Y583F | R438V | K512A |
| S36H/A37H | T263L | C403A | R438V/G446R | R531A |
| G45A | T263V | C403A/C589A | T445I | A534F |
| G45L | D307A | C403A/C589A/S36H/A37H | A465D | C589A |
| G45V | K324D | C403A/C589A/K46A | N467D | C589A/C34S |
| K46A | K324D/A534V | C403A/C589A/K82A | K482A | C589A/S36H/A37H |
| R59A/K60A | K324N | C403A/C589A/K94A/R95A/K96A | S486A | C589S |
| V78L | K368A | C403A/C589A/K368A | A492L | C589T |
| K82A | K368A/K46A | C403T | A492V | G733A |
| K94A/R95A/K96A | K368A/K82A | M414F | A495I | G733V |
| M213Q | K368A/K94A/R95A/K96A | M414I | R500A | |
| A231F | G369A | M414I/K555R | A501I | |

9. The protein variant of claim 8, comprising any one of the amino acid substitutions selected from the group consisting of G733A, G45A, T263I, T263V, K368A, K368A/K94A/R95A/K96A, C403A, C403A/C589A, C403A/C589A/S36H/A37H, C403A/C589A/K82A, C403A/C589A/K94A/R95A/K96A, C403A/C589A/K368A, C589A, C589A/S36H/A37H, C589S, C589T and G733V

10. The protein variant of claim 8, comprising any one of the amino acid substitutions selected from the group consisting of G733A, S36H/A37H, K94A/R95A/K96A, M213Q, M414F, T445I, S486A and A492L.

11. The protein variant of claim 8, comprising an amino acid substitution of G733A.

12. The protein variant of claim 4, comprising C403A/C589A amino acid substitution and further comprising at least one or more of amino acid substitutions selected from the group consisting of Q43C, R59C, R95C, K99C, S107C, A119C, K138C, T142C, A158C, K161C, A164C, A189C, K193C, N203C, R220C, K258C, S311C, A332C, S358C, K363C, E379C, K409C, K416C, S464C, R468C, Q523C, A530C, Q562C, D575C, Q585C, T590C, S596C, A610C, A627C, K634C, A651C, S658C, K700C, E710C and S745C.

13. The protein variant of claim 12, comprising any one of the amino acid substitutions selected from the group consisting of:
| | | | |
|---|---|---|---|
| Q43C/C403A/C589A | A164C/C403A/C589A | E379C/C403A/C589A | C403A/C589A/T590C |
| R59C/C403A/C589A | A189C/C403A/C589A | C403A/K409C/C589A | C403A/C589A/S596C |
| R95C/C403A/C589A | K193C/C403A/C589A | C403A/K416C/C589A | C403A/C589A/A610C |
| K99C/C403A/C589A | N203C/C403A/C589A | C403A/S464C/C589A | C403A/C589A/A627C |
| S107C/C403A/C589A | R220C/C403A/C589A | C403A/R468C/C589A | C403A/C589A/K634C |
| A119C/C403A/C589A | K258C/C403A/C589A | C403A/Q523C/C589A | C403A/C589A/A651C |
| K138C/C403A/C589A | S311C/C403A/C589A | C403A/A530C/C589A | C403A/C589A/S658C |
| T142C/C403A/C589A | A332C/C403A/C589A | C403A/Q562C/C589A | C403A/C589A/K700C |
| A158C/C403A/C589A | S358C/C403A/C589A | C403A/D575C/C589A | C403A/C589A/E710C |
| K161C/C403A/C589A | K363C/C403A/C589A | C403A/Q585C/C589A | C403A/C589A/S745C |

14. The protein variant of claim 12, which is Cys-specific PEGylated.

15. The protein variant of any one of claims 2 to 14, **characterized in that** one or more of GLP-1 inducibility, thermal stability and enzyme resistance is increased compared to the protein variant comprising the amino acid sequence of SEQ ID NO: 2.

16. A polynucleotide encoding the protein variant according to any one of claims 1 to 14.

17. A vector comprising the polynucleotide of claim 16.

18. A host cell comprising the vector of claim 17.

19. A pharmaceutical composition for reducing body weight and/or body fat, suppressing appetite, or treating or preventing metabolic diseases, comprising the protein variant according to any one of claims 1 to 14 and a pharmaceutically acceptable carrier.

20. The pharmaceutical composition of claim 19, wherein the metabolic diseases are selected from the group consisting of metabolic syndrome, obesity, insulin resistance, glucose tolerance, diabetes, arteriosclerosis, dyslipidemia, hyperlipidemia, hypercholesterolemia, fatty liver, cardiovascular disease, hypertension and osteoporosis.

21. A food composition for reducing body weight and/or body fat, suppressing appetite, or improving or preventing metabolic diseases, comprising the protein variant according to any one of claims 1 to 14.

22. The food composition of claim 21, wherein the metabolic diseases are selected from the group consisting of metabolic syndrome, obesity, insulin resistance, glucose tolerance, diabetes, arteriosclerosis, dyslipidemia, hyperlipidemia, hypercholesterolemia, fatty liver, cardiovascular disease, hypertension and osteoporosis.
